(19) Europäisches Patentamt
European Patent Office
Office européen des brevets

(11) EP 1 206 444 B1

(12) # EUROPEAN PATENT SPECIFICATION

(45) Date of publication and mention of the grant of the patent:
**12.11.2003 Bulletin 2003/46**

(21) Application number: **00953198.9**

(22) Date of filing: **23.08.2000**

(51) Int Cl.[7]: **C07C 251/24**, C07D 213/50, C07D 207/34, C07D 241/04, C07D 295/14, C07D 231/14, C07D 209/14, A61K 31/135, A61K 31/395

(86) International application number:
**PCT/EP00/08238**

(87) International publication number:
**WO 01/014320 (01.03.2001 Gazette 2001/09)**

(54) **COMPOUNDS THAT INHIBIT TRYPTASE ACTIVITY**

VERBINDUNGEN DIE DIE TRYPTASE-ACTIVITÄT HEMMEN

COMPOSES INHIBITEURS DE L'ACTION DE LA TRYPTASE

(84) Designated Contracting States:
**AT BE CH CY DE DK ES FI FR GB GR IE IT LI LU MC NL PT SE**

(30) Priority: **23.08.1999 DE 19939910**

(43) Date of publication of application:
**22.05.2002 Bulletin 2002/21**

(73) Proprietor: **Morphochem AG**
**81379 München (DE)**

(72) Inventors:
- **WEBER, Lutz**
  **81379 München (DE)**
- **FUCHS, Thilo**
  **81379 München (DE)**
- **ILLGEN, Katrin**
  **81379 München (DE)**
- **DOEMLING, Alexander**
  **81379 München (DE)**
- **CAPPI, Michael**
  **81379 München (DE)**

(74) Representative:
**Forstmeyer, Dietmar, Dr. rer. nat., Dipl.-Chem. et al**
**Boeters & Bauer,**
**Bereiteranger 15**
**81541 München (DE)**

(56) References cited:
**EP-A- 0 893 437**
**WO-A-94/20527**
**DE-A- 19 851 299**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 206 444 B1

## Description

The present invention relates to novel compounds, their pharmacologically acceptable salts, or solvates and hydrates, respectively, and to pharmaceutical compositions containing the same as active ingredient that are capable to inhibit tryptase activity in vivo. These novel compounds are potent tryptase inhibitors that make them useful in the prevention and/or treatment of diseases where tryptase is involved such as allergic diseases, inflammatory disorders such as asthma, rheumatoid arthritis and psoriasis. Also encompassed by the invention are processes for preparing such compounds, salts and compositions and the use thereof for the prevention and/or treatment of such diseases. The present invention furthermore relates to optically active forms, racemates and diastereomers of such compounds and salts, and to pro-drugs are composed of a compound of formula (I) and at least one pharmacologically acceptable protective group which will be cleared off under physiological conditions, selected from an alkoxy-, aralkyloxy-, acyl- or acyloxy group such as ethoxy, benzyloxy, acetyl or acetyloxy (herein below referred to as "pro-drugs");

Tryptases are a family of homologous serine proteases that are especially abundant in mast cells in a tetrameric complex with sulfated carbohydrates such as heparin. Upon activation of mast cells, catalytically active tryptase is released from the mast cells into extracellular fluids.

A series of diseases and disease states are related to the proteolytic activity of tryptase, which is involved in the activation of a series of other proteins like cytokines and enzymes that are in turn involved in such diseases. Therefore, the novel compounds of this invention, that are tryptase inhibitors, are useful in the treatment and/or prevention of a series of other diseases either by using them alone or in combination with other therapeutically useful agents. These diseases include or may include: inflammatory diseases of the pulmonary system like asthma, allergic rhinitis, chronic obstructive pulmonary disease, emphysema, viral and bacterial pulmonary infections and inflammatory responses (Kyle C. Elrod, Robert P. Numerof, Emerging Therapeutic Targets, 1999, 203-212).

Document WO 94 205 27 A refers to tryptase inhibitors useful for treating allergic and inflammatory disorders.

Other diseases where tryptase inhibitors may be of therapeutic use are rheumatoid arthritis, psoriasis, inflammatory bowel diseases, multiple sclerosis and cancer. Tryptase is abundant often in high concentrations in a variety of biological fluids and has a relatively long half-life.

It is an object of the present invention to provide novel compounds exhibiting useful properties, in particular tryptase-inhibiting activity.

More in detail, the object of the present invention is to provide new tryptase inhibitors having high activity and/or selectivity.

It is another object of the present invention to provide suitable pharmaceutical compositions. Said compounds and compositions, respectively, should be capable of being orally administered.

It is still another object of the present invention to provide a process for the preparation of these new compounds.

Moreover, it is desired that these new compounds are capable of being utilized in the prevention and/or treatment of diseases which involve tryptase activity.

The present invention describes compounds, their pharmacologically acceptable salts, or solvates and hydrates, respectively and formulations that are new and exhibit high activity and selectivity, and can be orally administered. The present invention furthermore relates to pro-drugs, optically active forms, racemates and diastereomers of such compounds and salts. These compounds and salts may, in turn, be pro-drugs which will be metabolically activated. The present invention furthermore describes pharmaceutical compositions containing said compounds and salts, respectively, as active ingredient. Furthermore, a straightforward and facile preparation of the compounds, pro-drugs, salts and compositions of the invention is disclosed as well as intermediates useful in such a synthesis, and the use of such active ingredients in the prevention and/or treatment of diseases which involve tryptase activity.

The present invention provides a compound of Formula (I):

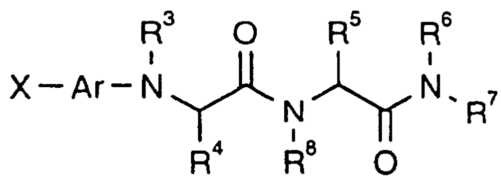

(I)

wherein

X is $H_2N-C(=NH)-$ or $R^1-N=C(-NH_2)-$, wherein

$R^1$ is -OH, $-C(=O)OR^2$, alkyl, aralkyl, aralkyloxy or a heteroalkyl group, such as alkyloxy, acyl or acyloxy, wherein

$R^2$ is alkyl, heteroalkyl, carbocyclic, heterocycloalkyl, aryl, heteroaryl or aralkyl;

Ar is arylene, heteroarylene, or aralkylene wherein X is directly attached to the aromatic ring system;

$R^3$ is H, alkyl, heteroalkyl or aralkyl;

$R^4$ is H, an alkyl group which may be substituted with one or more -OH or $-NH_2$ groups, a heteroalkyl group, a carbocyclic group, a heterocycloalkyl group, an aryl group, a heteroaryl group or an aralkyl group, which groups may be substituted with one or more groups selected from alkyl, heteroalkyl such as alkyloxy, acyl or acyloxy, a carbocyclic group, heterocycloalkyl, aryl, heteroaryl or aralkyl;

$R^5$ is H,.alkyl, heteroalkyl, carbocyclic, heterocycloalkyl, aryl, heteroaryl or aralkyl;

$R^6$ and $R^7$ are independently H, alkyl, heteroalkyl, carbocyclic, heterocycloalkyl such as aryl-heterocycloalkyl, aryl, heteroaryl, aralkyl or heteroarylalkyl, which groups may be substituted with one or more groups selected from alkyl, heteroalkyl such as alkoxy, acyl or acyloxy, a carbocyclic group, heterocycloalkyl, aryl, heteroaryl, aralkyl, -OH or $-NH_2$,

or are members of a heterocycloalkyl ring system, in particular an aryl-heterocycloalkyl ring system, or a heteroaryl ring system, which systems may be substituted with one or more groups selected from alkyl, heteroalkyl such as alkoxy, acyl or acyloxy, a carbocyclic group, heterocycloalkyl, aryl, heteroaryl, aralkyl, -OH or $-NH_2$; and

$R^8$ is H, alkyl, heteroalkyl, carbocyclic, heterocycloalkyl, aryl, heteroaryl or aralkyl;

or a pharmacologically acceptable salt, solvate or hydrate thereof.

The term alkyl refers to a saturated or unsaturated, straight or branched chain alkyl group, containing from one to ten carbon atoms preferably from one to six carbon atoms, for example methyl, ethyl, iso-propyl, iso-butyl, tert.-butyl, n-hexyl, 2,2-dimethylbutyl, n-octyl, allyl, isoprenyl or hexa-2-enyl groups.

The term heteroalkyl refers to an alkyl group where one or more carbon atoms are replaced by an oxygen, nitrogen, phosphorous or sulphur atom, for example an alkoxy group such as methoxy or ethoxy, or a methoxymethyl-, cyano- or 2,3-dioxyethyl group. The term heteroalkyl furthermore refers to a group derived from a carboxylic acid, and may, for example, be acyl, acyloxy, carboxyalkyl, carboxyalkyl ester, such as carboxyalkyl methyl ester, carboxyalkyl amide, alkoxycarbonyl or alkoxycarbonyloxy.

The term carbocyclic refers to a saturated or partially unsaturated, cyclic or branched cyclic group, having one or more rings, formed by a skeleton that contains from three to twelve carbon atoms, preferably from five or six to eight carbon atoms, for example cyclopropyl, cyclohexyl, tetralin or cyclohex-2-enyl groups.

The term heterocycloalkyl refers to a carbocyclic group where one or more carbon atoms are replaced by an oxygen, nitrogen, phosphorous or sulphur atom. Furthermore, a heterocycloalkyl group may be substituted by an alkyl, heteroalkyl or aryl group, and may, for example, be piperidino, morpholino, N-methyl-piperazino or N-phenyl-piperazino groups.

The term aryl refers to an aromatic cyclic or branched cyclic group, having one or more rings, formed by a skeleton that contains from three to twelve carbon atoms preferably from five or six to eight carbon atoms. Furthermore, an aryl group may be substituted by alkyl or heteroalkyl groups, and may, for example be a phenyl, naphthyl, 2-, 3- or 4-methoxyphenyl, 2-, 3- or 4-ethoxyphenyl, 4-carboxyphenyl alkyl or a 4-hydroxyphenyl group.

The term heteroaryl refers to an aryl group where one or more carbon atoms are replaced by an oxygen, nitrogen, phosphorous or sulphur atom, for example the 4-pyridyl, 2-imidazolyl, 3-pyrazolyl and isoquinolinyl groups.

The terms aralkyl and heteroarylalkyl refer to groups that comprise both aryl or, respectively, heteroaryl as well as alkyl and/or heteroalkyl and/or carbocyclic and/or heterocycloalkyl ring systems according to the above definitions, for example the tetrahydroisoquinolinyl, benzyl, 2-or 3-ethyl-indolyl or 4-methylpyridino groups.

The terms alkyl, heteroalkyl, carbocyclic, heterocycloalkyl, aryl, heteroaryl and aralkyl refer also to groups where one or more hydrogen atoms of such groups are replaced by fluorine, chlorine, bromine or iodine atoms. These terms furthermore refer to groups which are substituted with unsubstituted alkyl, heteroalkyl, aralkyl or aralkyloxy groups.

The terms arylene, heteroarylene and aralkylene refer to aryl-, heteroaryl- and aralkyl-groups which carry at least two substituents other than H.

Preferred are compounds of Formula (I) as defined above, wherein

X is $H_2N-C(=NH)-$ or $R^1-N=C(-NH_2)-$, wherein

$R^1$ is -OH or $-C(=O)OR^2$, wherein

$R^2$ is alkyl, heteroalkyl, carbocyclic, heterocycloalkyl, aryl, heteroaryl or aralkyl;

Ar is arylene, heteroarylene, or aralkylene;

$R^3$ is H, alkyl, heteroalkyl or aralkyl;
$R^4$ is H, alkyl which may be substituted with -OH or -$NH_2$ groups, heteroalkyl, carbocyclic groups, carboxyalkyl ester, heterocycloalkyl, aryl which may be substituted with acyl groups, heteroaryl or aralkyl;
$R^5$ is H, alkyl, heteroalkyl, carbocyclic, or aralkyl,
$R^6$ and. $R^7$ are independently H, alkyl, heteroalkyl, carbocyclic, heterocycloalkyl, aryl, heteroaryl, aralkyl which may be substituted with acyl groups or are members of the same heteroalkyl, cycloalkyl, heterocycloalkyl, aryl, heteroaryl which may be substituted with alkylene groups or aralkyl ring system, which may be substituted with -OH or -$NH_2$ groups, arylheterocycloalkyl, which may be substituted with acyl groups, heteroalkylaryl, which may be substitued with alkyl groups;
$R^8$ is H; or

or a pharmaceutically acceptable salt, solvate or hydrate thereof.

More preferred are compounds of Formula (I) as defined above wherein
X is $H_2N$-C(=NH)- or HO-N=C(-$NH_2$)- or $R^2$OC (=O)-N=C (-$NH_2$)-,
$R^3$ is H, Ar is meta-phenylene, and $R^5$ is a small alkyl or an aralkyl group; or compounds wherein
X is $H_2N$-C(=NH)- or HO-N=C(-$NH_2$)- or $R^2$OC(=0)-N=C(-$NH_2$)-,
$R^3$ is H, $R^4$ is H, methyl, hydroxymethyl, isopropyl, 2-imidazolyl, 3-pyrazolyl, Ar is meta-phenylene, $R^5$ is a small alkyl or an aralkyl group, and $R^8$ is H.

Especially preferred are those compounds of Formula I wherein
X-Ar are 3- or 4-methylenephenylamidimides, or 3- or 4-phenyleneamidimides or derivatives of the respective amidimide groups; or compounds wherein
X-Ar are 4-methylenephenylamidimide, or 3-phenylene-amidimide or derivatives of the respective amidimide groups.

Especially preferred are compounds as defined above,
wherein
X is $H_2N$-C(=NH)- or HO-N=C(-$NH_2$)- or $R^2$OC(=O)-N=C (-$NH_2$)-,
$R^3$ is H, $R^4$ is H, methyl, hydroxymethyl, 1,2-dihydroxyethyl, ethoxycarbonyl, isopropyl, cyclopropyl, 2-imidazolyl, 2-pyrrolyl, 3-pyrazolyl, 2-pyridyl, 4-methoxycarbonyl-phenyl, Ar is meta-phenylene, $R^5$ is a small alkyl or an aralkyl group, $R^6$ is H and $R^7$ is optionally substituted 1H-indol-3-yl-ethyl, 4-hydroxy-phenylethyl, cyclohexyl, N-(2-methoxyphenyl) piperazinyl, N-(4-methoxyphenyl)piperazinyl, 1,3-benzodioxol-5-ylmethyl, benzyl, phenethyl, 3,4-dimethoxyphenyl-1-ylmethyl, 2-methoxyphenyl-1-ylmethyl, 2-(4-morpholinyl)ethyl, 2-pyridinylethyl, 2-pyridinylpropyl, 3-pyridinylmethyl or $R^6$ and $R^7$ are part of a tetrahydroisoquinoline ring, a 4-thiomorpholine ring, a N-(2-methoxyphenyl)piperazine ring or a N-(4-methoxyphenyl)piperazine ring, and $R^8$ is H;
or wherein
X is $H_2N$-C(=NH)- or HO-N=C(-$NH_2$)- or $R^2$OC(=O)-N=C(-$NH_2$)-,
$R^3$ is H, Ar is a para-phenylmethylene group, $R^5$ is a small alkyl or an aralkyl group.

More preferred are compounds as defined above wherein
X is $H_2N$-C(=NH)- or HO-N=C(-$NH_2$)- or $R^2$OC(=O)-N=C(-$NH_2$)-,
$R^3$ is H, $R^4$ is H, methyl, hydroxymethyl, isopropyl, 2-imidazolyl, 3-pyrazolyl, Ar is para-phenylmethylene group, $R^5$ is a small alkyl or an aralkyl group;
or wherein
X is $H_2N$-C(=NH)- or HO-N=C (-$NH_2$)- or $R^2$OC(=O)-N=C (-$NH_2$)-,
$R^3$ is H, $R^4$ is H, methyl, hydroxymethyl, 1,2-dihydroxyethyl, ethoxycarbonyl, isopropyl, cyclopropyl, 2-imidazolyl, 2-pyrrolyl, 3-pyrazolyl, 3- or 4-phenoxy-phenyl, 1,3-benzodioxol-5-yl, 2-pyridyl, 4-methoxycarbonyl-phenyl, Ar is para-phenylmethylene group, $R^5$ is a small alkyl or an aralkyl group, $R^6$ is H and $R^7$ is optionally substituted 1H-indol-3-yl-ethyl, 4-hydroxy-phenethyl, cyclohexyl, N-(2-methoxyphenyl)piperazinyl, 1,3-benzodioxol-5-ylmethyl, benzyl, phenethyl, 3,4-dimethoxyphenyl-1-ylmethyl, 2-methoxyphenyl-1-ylmethyl, 2-(4-morpholinyl)ethyl, 2-pyridinylethyl, 2-pyridinylpropyl, 3-pyridinylmethyl or $R^6$ and $R^7$ are part of a tetrahydroisoquinoline ring, a 4-thiomorpholine ring, a N-(2-methoxyphenyl)piperazine ring or a N-(4-methoxyphenyl)-piperazine ring, and $R^8$ is H.

Further preferred compounds of Formula (I) are those compounds in which
$R^3$ is H;
further preferred compounds of Formula (I) are those compounds in which
$R^4$ is a small alkyl or carbocyclic group, or
$R^4$ is a small heteroalkyl group, or
$R^4$ is a small five membered heteroaryl group, or
$R^4$ is an aralkyl group;
further preferred compounds of Formula (I) are those compounds in which
$R^5$ is a small alkyl or carbocyclic group, or

$R^5$ is a small heteroalkyl group, or
$R^5$ is an aralkyl group;
further preferred compounds of Formula (I) are those compounds in which
$R^6$ is H, and
$R^7$ is an aralkyl group, or
$R^6$ and $R^7$ are members of the same aralkyl ring system;
further preferred compounds of Formula (I) are those compounds in which
$R^8$ is H;
further preferred compounds of Formula (I) are those compounds in which

$R^1$ is hydroxy, carboxy alkyl or carboxy heteroalkyl esters.

In the context of the present invention the term "small" refers to a group that contains up to 6 atoms such as, but not limited to, nitrogen, carbon or oxygen, not counting the number of hydrogen atoms.

The present invention also relates to pharmacologically acceptable salts, or solvates and hydrates, respectively, and to compositions of compounds of Formula (I). The present invention describes procedures to synthesize the above compounds, to produce pharmaceutically useful agents, which contain these compounds, as well as the use of these compounds for the production of pharmaceutically useful agents.

The pharmaceutical compositions according to the present invention contain at least one compound of Formula I as the active agent and optionally carriers and/or adjuvants.

Examples of such pharmacologically acceptable salts of compounds of Formula (I) are salts of physiologically acceptable mineral acids like hydrochloric, sulfuric and phosphoric acid; or salts of organic acids like methanesulfonic, p-toluenesulfonic, lactic, acetic, trifluoroacetic, citric, succinic, fumaric, maleinic and salicylic acid. Compounds of Formula (I) may be solvated, especially hydrated. The hydratisation can occur during the process of production or as a consequence of the hygroscopic nature of the initially water free compounds of Formula (I). The compounds of Formula (I) contain either none, one or two asymmetric C-atoms and may be present either as achiral compounds, mixtures of diastereomers, mixtures of enantiomers or as optically pure compounds.

The present invention also relates to pro-drugs which are composed of a compound of Formula (I) and at least one pharmacologically acceptable protective group which will be cleaved off under physiological conditions, selected from an alkoxy-, aralkyloxy-, acyl- or acyloxy group such as ethoxy, benzyloxy, acetyl or acetyloxy.

In a process for the preparation of a compound according to the present invention

a) a compound of Formula I, where X is a cyano group, is converted to a compound of Formula I, where X is a group of the Formula $R^1$-N=C($NH_2$)- or $H_2$N-C(=NH)-, and
b) this compound is optionally converted into a physiologically acceptable salt, solvate or hydrate.

The compounds of Formula (I) can moreover be synthesized e.g. by the conversion of a respective compound of Formula (I) where X is a cyano group (CN), into a compound of Formula I where X is an amidino group -C(=NH)$NH_2$ or the respective N-oxide of an amidino group -C(=N-OH)$NH_2$.

For the conversion of -CN into -C(=NH)$NH_2$ one can dissolve the starting nitrile in a solvent like ethanol or methanol or a solvent mixture as chloroform and methanol or chloroform and ethanol and expose this solution to a stream of water free hydrochloric acid at a temperature under 10 degrees Celsius. The intermediate product is precipitated with ether and filtered off after a reaction time of several hours to days. One can then dissolve this intermediate product in water, and extract it with a solvent like dichloromethane, chloroform or acetic acid ester after neutralisation with a base like sodium carbonate or hydroxide. The obtained material is then reacted with anhydrous ammonia or an ammonia salt like ammonia hydrochloride in a solvent like methanol or ethanol, preferentially at a temperature up to 80 degrees Celsius. Alternatively, one can react the filtered intermediate instantly with anhydrous ammonia or an ammonia salt like ammonia hydrochloride in a solvent like methanol or ethanol.

For the conversion of -CN to -C(=N-OH)$NH_2$ one can dissolve the starting nitrile in a solvent like dimethylformamide or ethanol and add the solution to a reaction mixture of a base like sodium, sodium hydride or triethylamine and hydroxylamine or a hydroxylamine salt like hydroxylamine hydrochloride in a solvent like dimethylformamide or ethanol, preferentially at a temperature below 5 degrees Celsius.

For the conversion of -CN to -C(=NH)$NH_2$ one can also first convert it to a compound -C(=N-OH)$NH_2$ according to the above procedure. In a second step this compound is than hydrogenated by dissolving it in a solvent like ethanol or acetic acid with a catalyst like palladium or palladium on charcoal or platinum or Raney-nickel under an atmosphere of hydrogen.

Compounds of Formula (I) where $R^1$ is -C(=O)O$R^2$ can be synthesized by reacting a compound of Formula (I) where $R^1$ is H, in a solvent like dimethylformamide or dichloromethane with a chloroformic acid ester of Formula ClC(=O)O$R^2$.

Compounds of Formula (I) where X is -CN or -C(=NH)$NH_2$ can be synthesized in one step by reacting an

amine of Formula (II), an aldehyde of Formula (III) and an isonitrile of Formula (IV)

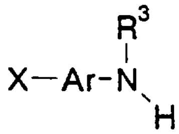

(II)

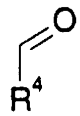

(III)

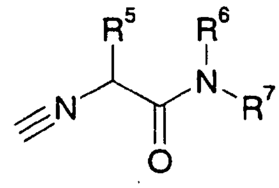

(IV)

in a solvent like methanol, iso-propanol, ethanol, dichloromethane or a mixture of solvents like methanol and water or iso-propanol and water. The described reaction can be catalysed by adding Brönsted acids like p-toluenesulfonic acid or 2,4-dinitrobenzene sulfonic acid or Lewis acids like zink dichloride, iron trichloride, boron trifluoro etherate or ytterbium triflate.

Compounds of Formula (I) where X is a cyano group serve as starting materials for the synthesis of the biologically active compounds described above. Compounds of Formula (I) where X is a cyano group can be synthesized according to methods known in general for forming amide bonds. Thus, an acid compound of Formula (V) and an amine compound of Formula (VI)

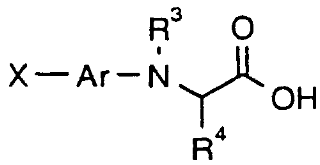

(V)

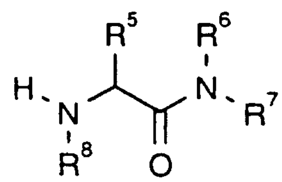

(VI)

can be coupled in a solvent like dimethylformamide with a coupling reagent like carbonyldiimidazole or dicyclohexylcarbodiimide and 1-hydroxybenztriazole.

Compounds of Formula (IV) can be synthesized by reacting an isonitrile of Formula (VII) with an amine of Formula (VIII)

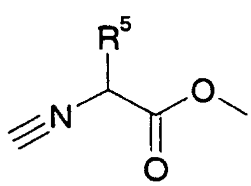

(VII)

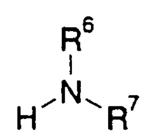

(VIII)

in a solvent like methanol, dichloromethane or dimethylformamide or without a solvent at room temperature or at a temperature up to 80 degrees Celsius (cf. K. Matsumoto et al., Synthesis, 1997, 249-50).

Compounds of Formula (V) can be synthesized by reacting an amine of Formula (IX) with an alpha-keto acid of Formula (X)

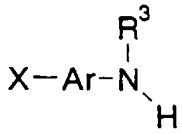 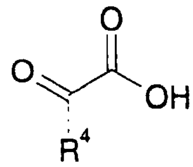

(IX) (X)

in a solvent like ethanol or methanol using sodium cyanoborohydride and catalytic amounts of acetic acid.

Alternatively, compounds of Formula (V) can be synthesized by reacting an alpha-bromo acid with a base like sodium hydroxide, evaporating the solvent and adding an excess of an amine of Formula (IX) and heating the resultant mixture at a preferred temperature of 80 to 120 degrees Celsius for a period of several hours.

Alternatively, compounds of Formula (V) can be synthesized by reacting an aldehyde like 4-cyanobenzaldehyde with an amino acid in an aqueous solution of a base like sodium hydroxide and adding sodium cyanoborohydride, preferentially at temperatures below 5 degrees Celsius.

Compounds of Formula (VI) can be synthesized by coupling an N-Boc protected amino acid with an amine of Formula (VIII) by using standard coupling methods with a coupling reagent like carbonyldiimidazole or dicyclohexylcarbodiimide and 1-hydroxybenzotriazole. Compounds of Formula (VI) can also be synthesized by using the mixed anhydrides or 4-nitrophenyl esters of the corresponding N-Boc protected amino acids. Deprotection of the amine group by treatment with an acid like hydrochloric acid in water or dichloromethane yields the final compounds of Formula (VI).

Compounds of Formula (VII) can be synthesized according to known procedures (I. Ugi editor, Isonitrile Chemistry in Organic Chemistry, Volume 20, Academic Press, 1971, New York and London).

A compound or a pharmaceutical composition or a pro-drug of the present invention can be used for the inhibition of tryptase, the treatment or prevention of diseases that are mediated by tryptase activity, for the treatment of allergic or inflammatory diseases, and especially for the treatment of asthma, allergic rhinitis, chronic obstructive pulmonary diseases, emphysema, viral and bacterial pulmonary infections and inflammatory responses, rheumatoid arthritis, multiple sclerosis, osteoarthritis, dermatological diseases, psoriasis, conjunctivitis, inflammatory bowel diseases, peptic ulcers, cardiovascular diseases, anaphylaxis and cancer.

To show the inhibition of the catalytic activity of tryptase one may use chromogenic peptide substrates. The inhibition of the amidolytic activity of tryptase by the compounds described above was shown as follows. The measurements were carried out at room temperature in microtiter plates. The compounds were dissolved in dimethylsulfoxide and 5 μl of this solution were added to a 2.8 nM solution of human recombinant tryptase in a Hepes buffer (pH: 7.8 In analogy to example 1 and using 100 mM Hepes, 140 mM NaCl, 0.1 % PEG 6000, 0.05 % Tween 80 and 200 nM heparin). Finally 750 μM of tosyl-glycyl-prolyl-lysine-4-nitranilide acetate in Hepes buffer were added and the hydrolysis of the substrate was followed with a spektrophometer. The same method but using N-methoxycarbonyl-D-norleucyl-glycyl-L-arginine-4-nitroanilide acetate as substrate was used to determine the inhibition of the proteolytic activity of factor Xa, i.e., another serin protease, by the compounds.

Some examples of compounds that inhibit tryptase with an $IC_{50}$ of below 90 nanomolar are given below:

| Compound from example | tryptase $IC_{50}$ (μM) | factor Xa $IC_{50}$ (μM) |
|---|---|---|
| 1 | < 0.09 | 5 |
| 2 | < 0.09 | 6.7 |
| 3 | < 0.09 | 21.9 |
| 4 | < 0.09 | 26.4 |
| 5 | < 0.09 | 5.7 |
| 6 | < 0.09 | > 110 |
| 7 | < 0.09 | > 110 |
| 8 | < 0.09 | 7.8 |
| 9 | < 0.09 | 2.4 |
| 10 | < 0.09 | 55 |
| 11 | < 0.09 | 4.45 |
| 12 | < 0.09 | 14.5 |
| 13 | < 0.09 | > 110 |

(continued)

| Compound from example | tryptase $IC_{50}$ (μM) | factor Xa $IC_{50}$ (μM) |
|---|---|---|
| 14 | < 0.09 | 7.15 |
| 15 | < 0.09 | 3.95 |
| 16 | < 0.09 | 9.2 |
| 17 | < 0.09 | 7.6 |
| 18 | < 0.09 | 8.6 |
| 19 | < 0.09 | 8.6 |
| 20 | < 0.09 | > 110 |
| 21 | < 0.09 | 7.7 |
| 22 | < 0.09 | 15 |
| 23 | < 0.09 | > 110 |
| 24 | < 0.09 | 1.6 |
| 25 | < 0.09 | 22.8 |

As mentioned above, therapeutically useful agents that contain compounds of Formula (I), their solvates and salts are also comprised in the scope of the present invention. In general, compounds of Formula (I) will be administered by using the known and acceptable modes known in the art, either alone or in combination with any other therapeutic agent. Such therapeutically useful agents can be administered by one of the following routes: oral, e.g. as dragees, coated tablets, pills, semisolids, soft or hard capsules, solutions, emulsions or suspensions, parenteral, e.g. as an injectable solution, rectal as suppositories, by inhalation, e.g. as a powder formulation or a spray, transdermal or intranasal. For the production of such tablets, pills, semisolids, coated tablets, dragees and hard gelatine capsules the therapeutically useful product may be mixed with pharmaceutically inert, inorganic or organic excipients as are e.g. lactose, sucrose, glucose, gelatin, malt, silica gel, starch or derivatives thereof, talc, stearinic acid or their salts or dried skim milk. For the production of soft capsules one may use excipients as are e.g. vegetable, petroleum, animal or synthetic oils, wax, fat, polyols. For the production of liquid solutions and syrups one may use excipients as are e.g. water, alcohols, aqueous saline, aqueous dextrose, polyols, glycerin, vegetable, petroleum, animal or synthetic oils. For suppositories one may use excipients as are e.g. vegetable, petroleum, animal or synthetic oils, wax, fat and polyols. For aerosol formulations one may use compressed gases suitable for this purpose, as are e.g. oxygen, nitrogen and carbon dioxide. The pharmaceutically useful agents may contain also additives for conservation, stabilisation, emulsifiers, sweetener, aromatisers, salts to change the osmotic pressure, buffers, coating additives and antioxidants.

Combinations with other therapeutic agents may include other therapeutically useful agents, e.g. that are used to prevent or treat asthma and allergic diseases, as are e.g. beta-adrenergic agonists, corticosteroids, methylxanthines, chromoglycates, leucotriene antagonists or histamine antagonists.

For the prevention and/or treatment of the diseases described above the dose of the biologically active compound may vary within broad limits and can be adjusted to the individual needs. In general a dose of 0.1 microgram to 4 milligram per kilogram body weight per day is appropriate, with a preferred dose of 0.5 to 1 or 2 milligram/kilogram per day. In appropriate cases the dose may be also higher or lower than given above.

**Examples**

Example 1: A 0.05 molar solution of glycolaldehyde, a 0.05 molar solution of 4-aminobenzamidine dihydrochloride and a 0.05 molar solution of N-[2-(1H-indol-3-yl)ethyl]-3-methylbutanamide-2-isonitrile in methanol was reacted for 24 hours at room temperature in a sealed vessel. After evaporation of the solvent the product was subjected to liquid chromatography and mass spectroscopy to verify the structural integrity of the final product. The product 2-{[2-({3-[amino(imino)-methyl]phenyl}amino)-3-hydroxypropanoyl]amino}-N-[2-(1H-indol-3-yl)ethyl]-3-methylbutanamide hydrochloride can be purified with liquid chromatography and using a water-methanol gradient as eluent on a reversed phase chromatography column. Calculated molweight: 465.2614 [M+H]+. Found ISP-TOF-MS: 465.3300 [M+H]+.

Example 2: In analogy to example 1 and using the corresponding appropriate starting materials 2-({3-[amino(imino)-methyl]phenyl}amino)-N-(2-{[2-(lH-indol-3-yl)ethyl]amino}-2-oxoethyl) acetamide hydrochloride was obtained. Calculated molweight: 393.2039 [M+H]+. Found ISP-TOF-MS: 393.2850 [M+H]+.

Example 3: In analogy to example 1 and using the corresponding appropriate starting materials 2-({3-[amino(imino)-methyl]phenyl}amino)-N-(2-{[2-(1H-indol-3-yl)ethyl]amino}-2-oxoethyl)propanamide hydrochloride was obtained. Calculated molweight: 407.2195 [M+H]+. Found ISP-TOF-MS: 407.2873 [M+H]+.

Example 4: In analogy to example 1 and using the corresponding appropriate starting materials 2-({3-[amino

(imino)-methyl]phenyl}amino)-N-(2-{[2-(1H-indol-3-yl)ethyl]amino}-2-oxoethyl)-2-(1H-pyrazol-3-yl)acetamide hydrochloride was obtained. Calculated molweight: 459.2257 [M+H]+. Found ISP-TOF-MS: 459.3132 [M+H]+.

Example 5: In analogy to example 1 and using the corresponding appropriate starting materials ethyl 2-({3-[amino-(imino)methyl]phenyl}amino)-3-{[1-({[2-(1H-indol-3-yl)ethyl]amino}carbonyl)-2-methylpropyl]amino}-3-oxopropanoate hydrochloride was obtained. Calculated molweight: 507.2720 [M+H]+. Found ISP-TOF-MS: 507.3644 [M+H]+.

Example 6: In analogy to example 1 and using the corresponding appropriate starting materials 2-({[({4-[amino (imino)-methyl]phenyl}methyl)amino]acetyl}amino)-N-{[3,4-bis(methyloxy)phenyl]methyl}propanamide hydrochloride was obtained. Calculated molweight: 428.2298 [M+H]+. Found ISP-TOF-MS: 428.2982 [M+H]+.

Example 7: In analogy to example 1 and using the corresponding appropriate starting materials methyl 4-(1-[({4-[amino(imino)methyl]phenyl}methyl)amino]-2-{[1-({[2-(1H-indol-3-yl)ethyl]amino}carbonyl)-2-methylpropyl] amino}-2-oxo-ethyl)benzoate hydrochloride was obtained. Calculated molweight: 583.3033 [M+H]+. Found ISP-TOF-MS: 583.3942 [M+H]+.

Example 8: In analogy to example 1 and using the corresponding appropriate starting materials 2-({3-[amino (imino)-methyl]phenyl)amino)-3-hydroxy-N-(2-{[2-(1H-indol-3-yl)ethyl]amino}-2-oxoethyl)propanamide hydrochloride was obtained. Calculated molweight: 423.2145 [M+H]+. Found ISP-TOF-MS: 423.2856 [M+H]+.

Example 9: In analogy to example 1 and using the corresponding appropriate starting materials 2-({3-[amino (imino)-methyl]phenyl}amino)-N-(2-{[2-(4-hydroxyphenyl)ethyl]amino}-2-oxoethyl)acetamide hydrochloride was obtained. Calculated molweight: 370.1879 [M+H]+. Found ISP-TOF-MS: 370.2571 [M+H]+.

Example 10: In analogy to example 1 and using the corresponding appropriate starting materials 2-({3-[amino (imino)-methyl]phenyl}amino)-N-(2-{[2- (1H-indol-3-yl)ethyl]amino}-2-oxoethyl)-3-methylbutanamide hydrochloride was obtained. Calculated molweight: 435.2508 [M+H]+. Found ISP-TOF-MS: 435.3217 [M+H]+.

Example 11: In analogy to example 1 and using the corresponding appropriate starting materials 2-{[({3-[amino (imino)methyl]phenyl}amino)acetyl]amino}-N-cyclohexyl-propanamide hydrochloride was obtained. Calculated molweight: 346.2243 [M+H]+. Found ISP-TOF-MS: 346.2902 [M+H]+.

Example 12: In analogy to example 1 and using the corresponding appropriate starting materials 2-{[({3-[amino (imino)methyl]phenyl}amino}acetyl]amino}-N-[2-(1H-in-dol-3-yl)ethyl]-3-methylbutanamide hydrochloride was obtained. Calculated molweight: 435.2508 [M+H]+. Found ISP-TOF-MS: 435.3305 [M+H]+.

Example 13: In analogy to example 1 and using the corresponding appropriate starting materials 2-[({4-[amino (imino)methyl]phenyl}methyl)amino]-N-(2-oxo-2-{[2-(2-pyridinyl)ethyl]amino}ethyl)propanamide hydrochloride was obtained. Calculated molweight: 383.2195 [M+H]+. Found ISP-TOF-MS: 383.2716 [M+H]+.

Example 14: In analogy to example 1 and using the corresponding appropriate starting materials 2-({3-[amino (imino)methyl]phenyl}amino)-N-{2-oxo-2-[(phenylmethyl)-amino]ethyl}acetamide hydrochloride was obtained. Calculated mol-weight: 340.1773 [M+H]+. Found ISP-TOF-MS: 340.2432 [M+H]+.

Example 15: In analogy to example 1 and using the corresponding appropriate starting materials 2-({3-[amino (imino)methyl]phenyl}amino)-N-[2-(3,4-dihydro-2(1H)-isoquinolinyl)-2-oxoethyl]-3-hydroxypropanamide hydrochloride was obtained. Calculated molweight: 396.2036 [M+H]+. Found ISP-TOF-MS: 396.2668 [M+H]+.

Example 16: In analogy to example 1 and using the corresponding appropriate starting materials 2-({3-[amino (imino)methyl]phenyl}amino)-N-[2-({[3,4-bis(methyloxy)-phenyl]methyl}amino)-2-oxoethyl]acetamide hydrochloride was obtained. Calculated molweight: 400.1985 [M+H]+. Found ISP-TOF-MS: 400.2699 [M+H]+.

Example 17: In analogy to example 1 and using the corresponding appropriate starting materials 2-{[2-({3-[amino(imino)methyl]phenyl}amino)-3-hydroxypropanoyl]amino}-3-methyl-N-(phenylmethyl)butanamide hydrochloride was obtained. Calculated molweight: 412.2349 [M+H]+. Found ISP-TOF-MS: 412.2932 [M+H]+.

Example 18: In analogy to example 1 and using the corresponding appropriate starting materials 2-{[2-({3-[amino (imino)methyl]phenyl}amino)-3-hydroxypropanoyl]amino}-N-(1,3-benzodioxol-5-ylmethyl)-3-methylbutanamide hydrochloride was obtained. Calculated molweight: 456.2247 [M+H]+. Found ISP-TOF-MS: 456.2862 [M+H]+.

Example 19: In analogy to example 1 and using the corresponding appropriate starting materials 2-{[2-({3-[amino(imino)methyl]phenyl}amino)-3-hydroxypropanoyl]amino}-N- (3,3-diphenylpropyl)-3-methylbutanamide hydrochloride was obtained. Calculated molweight: 516.2975 [M+H]+. Found ISP-TOF-MS: 516.3598 [M+H]+.

Example 20: In analogy to example 1 and using the corresponding appropriate starting materials 2-[({4-[amino (imino)methyl]phenyl}methyl)amino]-3-hydroxy-N-(2-oxo-2-{[2-(2-pyridinyl)ethyl]amino}ethyl)propanamide hydrochloride was obtained. Calculated molweight: 399.2145 [M+H]+. Found ISP-TOF-MS: 399.2585 [M+H]+.

Example 21: In analogy to example 1 and using the corresponding appropriate starting materials 2-[({4-[amino (imino)methyl]phenyl}methyl)amino]-N-(2-oxo-2-{[2-(2-pyridinyl)ethyl]amino}ethyl)-2-(1H-pyrrol-2-yl)acetamide hydrochloride was obtained. Calculated molweight: 434.2304 [M+H]+. Found ISP-TOF-MS: 434.2885 [M+H]+.

Example 22: In analogy to example 1 and using the corresponding appropriate starting materials 2-[({4-[amino (imino)methyl]phenyl}methyl)amino]-N-{2-oxo-2-[(3-pyridinylmethyl)amino]ethyl}-2-(1H-pyrrol-2-yl)acetamide hydrochloride was obtained. Calculated molweight: 420.2148 [M+H]+. Found ISP-TOF-MS: 420.2789 [M+H]+.

Example 23: In analogy to example 1 and using the corresponding appropriate starting materials 2-[({4-[amino

(imino)methyl]phenyl}methyl)amino]-N-(2- [4- (1,3-benzodioxol-5-ylmethyl)-1-piperazinyl]-2-oxoethyl}acetamide hydrochloride was obtained. Calculated molweight: 467.2407 [M+H]+. Found ISP-TOF-MS: 467.3090 [M+H]+.

Example 24: In analogy to example 1 and using the corresponding appropriate starting materials 2-({3-[amino (imino)-methyl]phenyl}amino)-N-(2-{4-[2-(methyloxy)phenyl]-1-piperazinyl}-2-oxoethyl)acetamide hydrochloride was obtained. Calculated molweight: 425.2301 [M+H]+. Found ISP-TOF-MS: 425.2956 [M+H]+.

Example 25: In analogy to example 1 and using the corresponding appropriate starting materials 2-({3-[amino (imino)-methyl]phenyl}amino)-N-(2-{[2-(4-morpholinyl)ethyl]amino}-2-oxoethyl)acetamide hydrochloride was obtained. Calculated molweight: 363.2145 [M+H]+. Found ISP-TOF-MS: 363.2838 [M+H]+.

Example 26: In analogy to example 1 and using the corresponding appropriate starting materials 2-[({4-[amino (imino)methyl]phenyl}methyl)amino]-N-(2-oxo-2-{[2-(2-pyridinyl)ethyl]amino}ethyl)-2-(1H-pyrazol-3-yl)acetamide hydrochloride was obtained. Calculated molweight: 435.2257 [M+H]+. Found ISP-TOF-MS: 435.2860 [M+H]+.

Example 27: In analogy to example 1 and using the corresponding appropriate starting materials methyl 4-{1-[({4-[amino(imino)methyl]phenyl}methyl)amino]-2-oxo-2-[(2-oxo-2-{[2-(2-pyridinyl)ethyl]amino}ethyl)amino]ethyl} benzoate hydrochloride was obtained. Calculated molweight: 503.2407 [M+H]+. Found ISP-TOF-MS: 503.3118 [M+H]+.

Example 28: In analogy to example 1 and using the corresponding appropriate starting materials methyl 4-[1-[({4-[amino(imino)methyl]phenyl}methyl)amino]-2-oxo-2-({2-oxo-2-[(3-pyridinylmethyl)amino]ethyl}amino)ethyl]benzoate hydrochloride was obtained. Calculated molweight: 489.2250 [M+H]+. Found ISP-TOF-MS: 489.2984 [M+H]+.

Example 29: In analogy to example 1 and using the corresponding appropriate starting materials ethyl 4-[({[2-({3-[amino(imino)methyl]phenyl}amino)-3-hydroxypropanoyl]amino}-acetyl) amino]-1-piperidinecarboxylate hydrochloride was obtained. Calculated molweight: 435.2356 (M+H]+. Found ISP-TOF-MS: 435.3009 (M+H]+.

Example 30: In analogy to example 1 and using the corresponding appropriate starting materials 2-({3-[amino (imino)-methyl]phenyl}amino)-3-hydroxy-N-(2-oxo-2-{[2-(2-pyridinyl)-ethyl]amino}ethyl)propanamide hydrochloride was obtained. Calculated molweight: 385.1988 [M+H]+. Found ISP-TOF-MS: 385.2572 [M+H]+.

Example 31: In analogy to example 1 and using the corresponding appropriate starting materials ethyl 2-({3-[amino(imino)methyl]phenyl}amino)-3-[(2-{[2-(1H-indol-3-yl)-ethyl]amino}-2-oxoethyl)amino]-3-oxopropanoate hydrochloride was obtained. Calculated molweight: 465.2250 [M+H]+. Found ISP-TOF-MS: 465.3121 [M+H]+.

Example 32: In analogy to example 1 and using the corresponding appropriate starting materials 2-{[2-({3-[amino(imino)methyl]phenyl}amino)-3-hydroxypropanoyl]amino}-3-methyl-N-[2-(2-pyridinyl)ethyl]butanamide hydrochloride was obtained. Calculated molweight: 427.2458 [M+H]+. Found ISP-TOF-MS: 427.3014 [M+H]+.

Example 33: In analogy to example 1 and using the corresponding appropriate starting materials 2-[({4-[amino (imino)methyl]phenyl}methyl)amino]-3-hydroxy-N-{2-oxo-2-[(3-pyridinylmethyl)amino]ethyl}propanamide hydrochloride was obtained. Calculated molweight: 385.1988 [M+H]+. Found ISP-TOF-MS: 385.2439 [M+H]+.

Example 34: In analogy to example 1 and using the corresponding appropriate starting materials 2-[({4-[amino (imino)methyl]phenyl}methyl)amino]-N-(2-{[2-(1H-indol-3-yl)ethyl]amino}-2-oxoethyl)propanamide hydrochloride was obtained. Calculated molweight: 421.2352 [M+H]+. Found ISP-TOF-MS: 421.2857 [M+H]+.

Example 35: In analogy to example 1 and using the corresponding appropriate starting materials 2-[({4-[amino (imino)methyl]phenyl}methyl)amino]-N-{2-oxo-2-[(3-pyridinylmethyl)amino]ethyl}-2-[4-(phenyloxy)phenyl]acetamide hydrochloride was obtained. Calculated molweight: 523.2458 [M+H]+. Found ISP-TOF-MS: 523.3289 [M+H]+.

Example 36: In analogy to example 1 and using the corresponding appropriate starting materials 2-({3-[amino (imino)-methyl]phenyl}amino) -N-[2-(3,4-dihydro-2 (1H) -isoquinolinyl)-2-oxoethyl]acetamide hydrochloride was obtained. Calculated molweight: 366.1930 [M+H]+. Found ISP-TOF-MS: 366.2608 [M+H]+.

Example 37: In analogy to example 1 and using the corresponding appropriate starting materials 2-({3-[amino (imino)-methyl)phenyl}amino)-N-(2-oxo-2-[(2-phenylethyl)amino]ethyl}acetamide hydrochloride was obtained. Calculated molweight: 354.1930 [M+H]+. Found ISP-TOF-MS: 354.2589 [M+H]+.

Example 38: In analogy to example 1 and using the corresponding appropriate starting materials 2-{[2-({3-[amino(imino)methyl]phenyl}amino)-3-hydroxypropanoyl]amino}-N-cyclohexyl-3-methylbutanamide hydrochloride was obtained. Calculated molweight: 404.2662 [M+H]+. Found ISP-TOF-MS: 404.3222 [M+H]+.

Example 39: In analogy to example 1 and using the corresponding appropriate starting materials 2-[({4-[amino (imino)methyl]phenyl}methyl)amino]-N-{2-oxo-2-{(4-pyridinylmethyl)amino]ethyl}-2-(1H-pyrrol-2-yl)acetamide hydrochloride was obtained. Calculated molweight: 420.2148 [M+H]+. Found ISP-TOF-MS: 420.2863 [M+H]+.

Example 40: In analogy to example 1 and using the corresponding appropriate starting materials 2-[({4-[amino (imino)methyl]phenyl}methyl)amino]-N-(2-oxo-2-{[1-(phenylmethyl)-4-piperidinyl]amino}ethyl)acetamide hydrochloride was obtained. Calculated molweight: 437.2665 [M+H]+. Found ISP-TOF-MS: 437.3293 [M+H]+.

Example 41: In analogy to example 1 and using the corresponding appropriate starting materials 1,1-dimethylethyl 2-[({[({3-[amino(imino)methyl]phenyl}amino)acetyl]amino}ace-tyl)amino] ethylcarbamate hydrochloride was obtained. Calculated molweight: 393.2250 [M+H]+. Found ISP-TOF-MS: 393.2945 [M+H]+.

Example 42: In analogy to example 1 and using the corresponding appropriate starting materials 2-{[({3-[amino

(imino)methyl]phenyl}amino)acetyl]amino}-N-(3,3-diphenylpropyl)propanamide hydrochloride was obtained. Calculated molweight: 458.2556 [M+H]+. Found ISP-TOF-MS: 458.3299 [M+H]+.

Example 43: In analogy to example 1 and using the corresponding appropriate starting materials 2-({3-[amino (imino)-methyl]phenyl}amino)-N-[2-(cycloxelamino)-2-oxoethyl]-3,4-dihydroxybutanamide hydrochloride was obtained. Calculated molweight: 392.2298 [M+H]+. Found ISP-TOF-MS: 392.3112 [M+H]+.

Example 44: In analogy to example 1 and using the corresponding appropriate starting materials 2-({3-[amino (imino)-methyl]phenyl}amino)-3-hydroxy-N-{2-oxo-2-[(3-pyridinylmethyl)amino]ethyl}propanamide hydrochloride was obtained. Calculated molweight: 371.1832 [M+H]+. Found ISP-TOF-MS: 371.2459 [M+H]+.

Example 45: In analogy to example 1 and using the corresponding appropriate starting materials 2-[({4-[amino (imino)methyl]phenyl}methyl)amino]-3-hydroxy-N-(2-{[2-(1H-indol-3-yl)ethyl]amino}-2-oxoethyl)propanamide hydrochloride was obtained. Calculated molweight: 437.2301 [M+H]+. Found ISP-TOF-MS: 437.2726 [M+H]+.

Example 46: In analogy to example 1 and using the corresponding appropriate starting materials 2-[({4-[amino (imino)methyl]phenyl}methyl)amino]-N-(2-{[2-(4-hydroxyphenyl)ethyl]amino}-2-oxoethyl)-2-(1H-pyrrol-2-yl)acetamide hydrochloride was obtained. Calculated molweight: 449.2301 [M+H]+. Found ISP-TOF-MS: 449.2949 [M+H]+.

Example 47: In analogy to example 1 and using the corresponding appropriate starting materials 2-({3-[amino (imino)-methyl]phenyl}amino)-3-hydroxy-N-[2-oxo-2-(1-piperidinylamino)ethyl]propanamide hydrochloride was obtained. Calculated molweight: 363.2145 [M+H]+. Found ISP-TOF-MS: 363.2780 [M+H]+.

Example 48: In analogy to example 1 and using the corresponding appropriate starting materials 2-({3-[amino (imino)-methyl]phenyl}amino)-3-hydroxy-N-[2-oxo-2-(4-thiomorpholinyl)ethyl]propanamide hydrochloride was obtained. Calculated molweight: 366.1600 [M+H]+. Found ISP-TOF-MS: 366.2160 [M+H]+.

Example 49: In analogy to example 1 and using the corresponding appropriate starting materials 2-{[2-({3-[amino(imino)methyl]phenyl}amino)-3-hydroxypropanoyl]amino}-N-{[3,4-bis(methyloxy)phenyl]methyl}-3-methylbutanamide hydrochloride was obtained. Calculated molweight: 472.2560 [M+H]+. Found ISP-TOF-MS: 472.3187 [M+H]+.

Example 50: In analogy to example 1 and using the corresponding appropriate starting materials 2-{[2-({3-[amino(imino)methyl]phenyl}amino)propanoyl]amino}-N-[2-(1H-indol-3-yl)ethyl]-3-methylbutanamide hydrochloride was obtained. Calculated molweight: 449.2665 [M+H]+. Found ISP-TOF-MS: 449.3438 [M+H]+.

Example 51: In analogy to example 1 and using the corresponding appropriate starting materials 2-{[({3-[amino (imino)methyl]phenyl}amino)acetyl]amino}-N-[2-(1H-in-dol-3-yl)ethyl]-3-methylbutanamide hydrochloride was obtained. Calculated molweight: 435.2508 [M+H]+. Found ISP-TOF-MS: 435.3112 [M+H]+.

Example 52: In analogy to example 1 and using the corresponding appropriate starting materials 2-({3-[amino (imino)-methyl]phenyl}amino)-3,4-dihydroxy-N-[2-oxo-2-(4-thiomorpholinyl)ethyl]butanamide hydrochloride was obtained. Calculated molweight: 396.1706 [M+H]+. Found ISP-TOF-MS: 396.2505 [M+H]+.

Example 53: In analogy to example 1 and using the corresponding appropriate starting materials 2-({3-[amino (imino)-methyl]phenyl}amino)-3,4-dihydroxy-N-(2-{4-[2-(methyloxy)-phenyl]-1-piperazinyl}-2-oxoethyl)butanamide hydrochloride was obtained. Calculated molweight: 485.2512 [M+H]+. Found ISP-TOF-MS: 485.3445 [M+H]+.

Example 54: In analogy to example 1 and using the corresponding appropriate starting materials 2-({3-[amino (imino)-methyl]phenyl}amino)-N-{1-[({[3,4-bis(methyloxy)phenyl]methyl}amino)carbonyl]-2-methylpropyl}-3,4-dihydroxybutanamide hydrochloride was obtained. Calculated molweight: 502.2666 [M+H]+. Found ISP-TOF-MS: 502.3630 [M+H]+.

Example 55: In analogy to example 1 and using the corresponding appropriate starting materials 2-[({4-[amino (imino)methyl]phenyl}methyl)amino]-N-{2-oxo-2-[(2-phenylethyl)amino]ethyl}-2-(2-pyridinyl)acetamide hydrochloride was obtained. Calculated molweight: 445.2352 [M+H]+. Found ISP-TOF-MS: 445.2903 [M+H]+.

Example 56: In analogy to example 1 and using the corresponding appropriate starting materials 2-[({4-[amino (imino)methyl]phenyl}methyl)amino]-N-(2-{[2-(4-hydroxyphenyl)ethyl]amino}-2-oxoethyl)-2-(2-pyridinyl)acetamide hydrochloride was obtained. Calculated molweight: 461.2301 [M+H]+. Found ISF-TOF-MS: 461.2852 [M+H]+.

Example 57: In analogy to example 1 and using the corresponding appropriate starting materials 1,1-dimethylethyl 2-[({[[({4-[amino(imino)methyl]phenyl}methyl)amino](2-pyridinyl)acetyl]amino}acetyl)amino]ethylcarbamate hydrochloride was obtained. Calculated molweight: 484.2672 [M+H]+. Found ISP-TOF-MS: 484.3214 [M+H]+.

Example 58: In analogy to example 1 and using the corresponding appropriate starting materials 2-({[({4-[amino(imino)methyl]phenyl}methyl)amino]acetyl}amino)-3-methyl-N-[2-(2-pyridinyl)ethyl]butanamide hydrochloride was obtained. Calculated molweight: 411.2508 [M+H]+. Found ISP-TOF-MS: 411.3163 [M+H]+.

Example 59: In analogy to example 1 and using the corresponding appropriate starting materials 2-({3-[amino (imino)-methyl]phenyl}amino)-3-hydroxy-N-{2-oxo-2-[(phenylmethyl)-amino]ethyl}propanamide hydrochloride was obtained. Calculated molweight: 370.1879 [M+H]+. Found ISP-TOF-MS: 370.2430 [M+H]+.

Example 60: In analogy to example 1 and using the corresponding appropriate starting materials 2-[({4-[amino (imino)methyl]phenyl}methyl)amino]-3-methyl-N-(2-oxo-2-{[2-(2-pyridinyl)ethyl]amino}ethyl)butanamide hydrochloride was obtained. Calculated molweight: 411.2508 [M+H]+. Found ISP-TOF-MS: 411.3092 [M+H]+.

Example 61: In analogy to example 1 and using the corresponding appropriate starting materials 2-[({4-[amino

(imino)methyl]phenyl]methyl}amino]-N-{2-oxo-2-[(4-pyridinylmethyl)amino]ethyl}-2-(1H-pyrazol-3-yl)acetamide hydrochloride was obtained. Calculated molweight: 421.2100 [M+H]+. Found ISP-TOF-MS: 421.2773 [M+H]+.

Example 62: In analogy to example 1 and using the corresponding appropriate starting materials 2-({3-[amino (imino)-methyl]phenyl}amino)-3-hydroxy-N-{2-oxo-2-[(4-pyridinylmethyl)amino]ethyl}propanamide hydrochloride was obtained. Calculated molweight: 371.1832 [M+H]+. Found ISP-TOF-MS: 371.2456 [M+H]+.

Example 63: In analogy to example 1 and using the corresponding appropriate starting materials 2-({3-[amino (imino)-methyl]phenyl}amino)-N-{2-[(3,3-diphenylpropyl}amino]-2-oxo-ethyl}-3-hydroxypropanamide hydrochloride was obtained. Calculated molweight: 474.2505 [M+H]+. Found ISP-TOF-MS: 474.3172 [M+H]+.

Example 64: In analogy to example 1 and using the corresponding appropriate starting materials 2-[({4-[amino (imino)methyl]phenyl}methyl)amino]-3-hydroxy-N-(2-oxo-2-{[1-(phenylmethyl)-4-piperidinyl]amino}ethyl)propanamide hydrochloride was obtained. Calculated molweight: 467.2771 [M+H]+. Found ISP-TOF-MS: 467.3214 [M+H]+.

Example 65: In analogy to example 1 and using the corresponding appropriate starting materials 2-({3-[amino (imino)-methyl]phenyl}amino)-3-hydroxy-N-[2-oxo-2-(1,2,3,4-tetra-hydro-1-naphthalenylamino)ethyl]propanamide hydrochloride was obtained. Calculated molweight: 410.2192 [M+H]+. Found ISP-TOF-MS: 410.2775 [M+H]+.

Example 66: In analogy to example 1 and using the corresponding appropriate starting materials 2-({3-[amino (imino)-methyl]phenyl}amino)-N-[2-(3,4-dihydro-2(1H)-isoquinolinyl)-2-oxoethyl]propanamide hydrochloride was obtained. Calculated molweight: 380.2086 [M+H]+. Found ISP-TOF-MS: 380.2714 [M+H]+.

Example 67: In analogy to example 1 and using the corresponding appropriate starting materials 2-({3-[amino (imino)-methyl]phenyl}amino)-N-(2-{[2-(4-morpholinyl)ethyl]amino}-2-oxoethyl)-2-(2-pyridinyl)acetamide hydrochloride was obtained. Calculated molweight: 440.2410 [M+H]+. Found ISP-TOF-MS: 440.3192 [M+H]+.

Example 68: In analogy to example 1 and using the corresponding appropriate starting materials 2-({3-[amino (imino)-methyl]phenyl}amino)-N-{2-[4-(1,3-benzodioxol-5-ylmethyl)-1-piperazinyl]-2-oxoethyl)acetamide hydrochloride was obtained. Calculated molweight: 453.2250 [M+H]+. Found ISP-TOF-MS: 453.2939 [M+H]+.

Example 69: In analogy to example 1 and using the corresponding appropriate starting materials 2-{[({3-[amino (imino)methyl]phenyl}amino)acetyl]amino}-3-methyl-N-[2-(2-pyridinyl)ethyl]butanamide hydrochloride was obtained. Calculated molweight: 397.2352 [M+H]+. Found ISP-TOF-MS: 397.3010 [M+H]+.

Example 70: In analogy to example 1 and using the corresponding appropriate starting materials 2-{[({3-[amino (imino)methyl]phenyl}amino)acetyl]amino}-N-(3,3-di-phenylpropyl)-3-methylbutanamide hydrochloride was obtained. Calculated molweight: 486.2869 [M+H]+. Found ISP-TOF-MS: 486.3620 [M+H]+.

Example 71: In analogy to example 1 and using the corresponding appropriate starting materials 2-({3-[amino (imino)-methyl]phenyl}amino)-3,4-dihydroxy-N-[2-({[2-(methyloxy)-phenyl]methyl}amino)-2-oxoethyl]butanamide hydrochloride was obtained. Calculated molweight: 430.2090 [M+H]+. Found ISP-TOF-MS: 430.2946 [M+H]+.

Example 72: In analogy to example 1 and using the corresponding appropriate starting materials 2-({3-[amino (imino)-methyl)phenyl)amino)-N-[2-({2- [3,4-bis(methyloxy)phenyl]-ethyl}amino)-2-oxoethyl]-3,4-dihydroxybutanamide hydrochloride was obtained. Calculated molweight: 474.2353 [M+H]+. Found ISP-TOF-MS: 474.3280 [M+H]+.

Example 73: In analogy to example 1 and using the corresponding appropriate starting materials 2-({3-[amino (imino)-methyl]phenyl}amino)-N-(1-{[(3,3-diphenylpropyl)amino]-carbonyl}-2-methylpropyl)-3,4-dihydroxybutanamide hydrochloride was obtained. Calculated molweight: 546.3080 [M+H]+. Found ISP-TOF-MS: 546.4065 [M+H]+.

Example 74: In analogy to example 1 and using the corresponding appropriate starting materials 2-({3-[amino (imino)-methyl]phenyl}amino)-N-{2-[4-(1,3-benzodioxol-5-ylmethyl)-1-piperazinyl]-2-oxoethyl}-3-hydroxypropanamide hydrochloride was obtained. Calculated molweight: 483.2356 [M+H]+. Found ISP-TOF-MS: 483.2940 [M+H]+.

Example 75: In analogy to example 1 and using the corresponding appropriate starting materials 2-({3-[amino (imino)-methyl]phenyl}amino)-N-[2-({[3,4-bis(methyloxy)phenyl]-methyl}amino)-2-oxoethyl]-3-hydroxypropanamide hydrochloride was obtained. Calculated molweight: 430.2090 [M+H]+. Found ISP-TOF-MS: 430.2743 [M+H]+.

Example 76: In analogy to example 1 and using the corresponding appropriate starting materials 2-[({4-[amino (imino)methyl]phenyl}methyl)amino]-N-[2-({[3,4-bis(methyloxy)phenyl]methyl}amino)-2-oxoethyl]-2-(2-pyridinyl) acetamide hydrochloride was obtained. Calculated mol-weight: 491.2407 [M+H]+. Found ISP-TOF-MS: 491.2984 [M+H]+.

Example 77: In analogy to example 1 and using the corresponding appropriate starting materials 2-[({4-[amino (imino)methyl]phenyl}methyl)amino]-N-[2-(cyclohexylamino)-2-oxoethyl]-2-(2-pyridinyl)acetamide hydrochloride was obtained. Calculated molweight: 423.2508 [M+H]+. Found ISP-TOF-MS: 423.3087 [M+H]+.

Example 78: In analogy to example 1 and using the corresponding appropriate starting materials 2-({[({4-[amino(imino)methyl]phenyl}methyl)amino]acetyl}amino)-N-[2-(1H-indol-3-yl)ethyl]-3-methylbutanamide hydrochloride was obtained. Calculated molweight: 449.2665 [M+H]+. Found ISP-TOF-MS: 449.3335 [M+H]+.

Example 79: In analogy to example 1 and using the corresponding appropriate starting materials 2-({3-[amino (imino)-methyl]phenyl}amino)-3-hydroxy-N-{2-oxo-2-[4-(2-pyrimidinyl)-1-piperazinyl]ethyl}propanamide hydrochloride was obtained. Calculated molweight: 427.2206 [M+H]+. Found ISP-TOF-MS: 427.2853 [M+H]+.

Example 80: In analogy to example 1 and using the corresponding appropriate starting materials 2-({3-[amino

(imino)-methyl]phenyl}amino)-3,4-dihydroxy-N-(2-{[2-(1H-indol-3-yl)-ethyl]amino}-2-oxoethyl)butanamide hydrochloride was obtained. Calculated molweight: 453.2250 [M+H]+. Found ISP-TOF-MS: 453.3141 [M+H]+.

Example 81: In analogy to example 1 and using the corresponding appropriate starting materials 2-({3-[amino (imino)-methyl]phenyl}amino)-3-hydroxy-N-(2-{4-[2-(methyloxy)-phenyl]-1-piperazinyl}-2-oxoethyl)propanamide hydrochloride was obtained. Calculated molweight: 455.2407 [M+H]+. Found ISP-TOF-MS: 455.2971 [M+H]+.

Example 82: In analogy to example 1 and using the corresponding appropriate starting materials 2-({3-[amino (imino)-methyl]phenyl}amino)-N-[2-(cyclohexylamino)-2-oxoethyl]-acetamide hydrochloride was obtained. Calculated molweight: 332.2086 [M+H]+. Found ISP-TOF-MS: 332.2682 (M+H]+.

Example 83: In analogy to example 1 and using the corresponding appropriate starting materials 2-[({4-[amino (imino)methyl]phenyl}methyl)amino)-N-(2-oxo-2-{[2-(2-pyridinyl)ethyl]amino)ethyl)acetamide hydrochloride was obtained. Calculated molweight: 369.2039 [M+H]+. Found ISP-TOF-MS: 369.2658 [M+H]+.

Example 84: In analogy to example 1 and using the corresponding appropriate starting materials 2-({3-[amino (imino)-methyl]phenyl}amino)-3-hydroxy-N-(2-{[2- (4-hydroxyphenyl)-ethyl]amino}-2-oxoethyl)propanamide hydrochloride was obtained. Calculated molweight: 400.1985 [M+H]+. Found ISP-TOF-MS: 400.2646 [M+H]+.

Example 85: In analogy to example 1 and using the corresponding appropriate starting materials 2-({3-[amino (imino)-methyl]phenyl}amino)-N-[2-oxo-2-(1-piperidinylamino)ethyl]-acetamide hydrochloride was obtained. Calculated molweight: 333.2039 [M+H]+. Found ISP-TOF-MS: 333.2659 [M+H]+.

Example 86: In analogy to example 1 and using the corresponding appropriate starting materials 2-({3-[amino (imino)-methyl]phenyl}amino)-N-{2-[(diphenylmethyl)amino]-2-oxo-ethyl}-3,4-dihydroxybutanamide hydrochloride was obtained. Calculated molweight: 476.2298 [M+H]+. Found ISP-TOF-MS: 476.3239 [M+H]+.

Example 87: In analogy to example 1 and using the corresponding appropriate starting materials 2-[({4-[amino (imino)methyl]phenyl}methyl)amino]-N-(2-oxo-2-{[2-(2-pyridinyl)ethyl]amino}ethyl)acetamide hydrochloride was obtained. Calculated molweight: 369.2039 [M+H]+. Found ISP-TOF-MS: 369.2478 [M+H]+.

Example 88: In analogy to example 1 and using the corresponding appropriate starting materials 2-[({4-[amino (imino)methyl]phethyl}methyl)amino]-N-(2-{[2-(1H-indol-3-yl)ethyl]amino}-2-oxoethyl)acetamide hydrochloride was obtained. Calculated molweight: 407.2195 [M+H]+. Found ISP-TOF-MS: 407.2801 [M+H]+.

Example 89: In analogy to example 1 and using the corresponding appropriate starting materials 2-({3-[amino (imino)-methyl]phenyl}amino)-3,4-dihydroxy-N-[2-oxo-2-(1-piperidinylamino)ethyl]butanamide hydrochloride was obtained. Calculated molweight: 393.2250 [M+H]+. Found ISP-TOF-MS: 393.3045 [M+H]+.

Example 90: In analogy to example 1 and using the corresponding appropriate starting materials 2-{[({3-[amino (imino)methyl]phenyl}amino)acetyl]amino}-N-{[3,4-bis-(methyloxy)phenyl]methyl}propanamide hydrochloride was obtained. Calculated molweight: 414.2141 [M+H]+. Found ISP-TOF-MS: 414.2859 [M+H]+.

Example 91: In analogy to example 1 and using the corresponding appropriate starting materials 2-[({4-[amino (imino)methyl]phenyl}methyl)amino]-3,4-dihydroxy-N-(2-oxo-2-{[2-(2-pyridinyl)ethyl]amino}ethyl)butanamide hydrochloride was obtained. Calculated molweight: 429.2250 [M+H]+. Found ISP-TOF-MS: 429.2883 [M+H]+.

Example 92: In analogy to example 1 and using the corresponding appropriate starting materials 2-({3-[amino (imino)-methyl]phenyl}amino)-N- [2-(3,4-dihydro-2(1H)-isoquinolinyl)-2-oxoethyl]-3,4-dihydroxybutanamide hydrochloride was obtained. Calculated molweight: 426.2141 [M+H]+. Found ISP-TOF-MS: 426.2958 [M+H]+.

Example 93: In analogy to example 1 and using the corresponding appropriate starting materials 2-[({4-[amino (imino)methyl]phenyl}methyl)amino]-3-hydroxy-N-[2-oxo-2-(1-piperidinylamino)ethyl]propanamide hydrochloride was obtained. Calculated molweight: 377.2301 [M+H]+. Found ISP-TOF-MS: 377.2749 [M+H]+.

Example 94: In analogy to example 1 and using the corresponding appropriate starting materials methyl 4-[1'-[({4-[amino(imino)methyl]phenyl}methyl)amino]-2-oxo-2-({2-oxo-2-[(4-pyridinylmethyl)amino]ethyl}amino)ethyl] benzoate hydrochloride was obtained. Calculated molweight: 489.2250 [M+H]+. Found ISP-TOF-MS: 489.2938 [M+H] +.

Example 95: In analogy to example 1 and using the corresponding appropriate starting materials 2-[({4-[amino (imino)methyl]phenyl}methyl)amino]-N-{2-oxo-2-[(phenylmethyl)amino)ethyl}-2-(2-pyridinyl)acetamide hydrochloride was obtained. Calculated molweight: 431.2195 [M+H]+. Found ISP-TOF-MS: 431.2820 [M+H]+.

Example 96: In analogy to example 1 and using the corresponding appropriate starting materials 2-({3-[amino (imino)-methyl]phenyl}amino)-3,4-dihydroxy-N-(2-oxo-2-{[2-(2-pyridinyl)ethyl]amino}ethyl)butanamide hydrochloride was obtained. Calculated molweight: 415.2094 [M+H]+. Found ISP-TOF-MS: 415.2910 [M+H]+.

Example 97: In analogy to example 1 and using the corresponding appropriate starting materials 2-({3-[amino (imino)-methyl]phenyl}amino)-N-{2-[(3,3-diphenylpropyl)amino]-2-oxo-ethyl}-3,4-dihydroxybutanamide hydrochloride was obtained. Calculated molweight: 504.2611 [M+H]+. Found ISP-TOF-MS: 504.2836 [M+H]+.

Example 98: In analogy to example 1 and using the corresponding appropriate starting materials 2-({3-[amino (imino)-methyl]phenyl}amino)-3-hydroxy-N-(2-oxo-2-{[1-(phenylmethyl)-4-piperidinyl]amino}ethyl)propanamide hydrochloride was obtained. Calculated molweight: 453.2614 [M+H]+. Found ISP-TOF-MS: 453.3273 [M+H]+.

Example 99: In analogy to example 1 and using the corresponding appropriate starting materials 2-({3-(amino

(imino)-methyl]phenyl}amino)-N-[2-oxo-2-(4-thiomorpholinyl)ethyl]-acetamide hydrochloride was obtained. Calculated molweight: 336.1494 [M+H]+. Found ISP-TOF-MS: 336.2139 [M+H]+.

Example 100: In analogy to example 1 and using the corresponding appropriate starting materials 2-[({4-[amino(imino)methyl]phenyl}methyl)amino]-3-hydroxy-N-{2-oxo-2-[4-(2-pyrimidinyl)-1-piperazinyl]ethyl}propanamide hydrochloride was obtained. Calculated molweight: 441.2363 [M+H]+. Found ISP-TOF-MS: 441.2844 [M+H]+.

Example 101: In analogy to example 1 and using the corresponding appropriate starting materials 2-[({4-[amino(imino)methyl]phenyl}methyl)amino]-2-(1,3-benzodioxol-5-yl)-N-(2-oxo-2-{[2-(2-pyridinyl)ethyl]amino}ethyl)acetamide hydrochloride was obtained. Calculated molweight: 489.2250 [M+H]+. Found ISP-TOF-MS: 489.2841 [M+H]+.

Example 102: In analogy to example 1 and using the corresponding appropriate starting materials 2-({2-[({4-[amino(imino)methyl]phenyl}methyl)amino]-3-hydroxypropanoyl}amino)-3-methyl-N- [2- (2-pyridinyl)ethyl]butanamide hydrochloride was obtained. Calculated molweight: 441.2614 [M+H]+. Found ISP-TOF-MS: 441.3016 [M+H]+.

Example 103: In analogy to example 1 and using the corresponding appropriate starting materials 2-({3-[amino (imino)-methyl]phenyl}amino)-3-hydroxy-N-{2-oxo-2-[(2-phenylethyl)-amino]ethyl}propanamide hydrochloride was obtained. Calculated molweight: 384.2036 [M+H]+. Found ISP-TOF-MS: 384.2638 [M+H]+. Example 104: In analogy to example 1 and using the corresponding appropriate starting materials 2-[({4-[amino(imino)methyl]phenyl}methyl)amino]-2-(1,3-benzodioxol-5-yl)-N-{2-oxo-2-[(3-pyridinylmethyl)amino]ethyl}acetamide hydrochloride was obtained. Calculated molweight: 475.2094 [M+H]+. Found ISP-TOF-MS: 475.2714 [M+H]+.

Example 105: In analogy to example 1 and using the corresponding appropriate starting materials 2-({3-[amino (imino)-methyl]phenyl}amino)-N-(2-{[2-(IH-indol-3-yl)ethyl]amino}-2-oxoethyl)acetamide hydrochloride was obtained. Calculated molweight: 393.2039 [M+H]+. Found ISP-TOF-MS: 393.2678 [M+H]+.

Example 106: In analogy to example 1 and using the corresponding appropriate starting materials ethyl 4-[({[({3-[amino(imino)methyl]phenyl}amino)acetyl]amino}acetyl)amino]-1-piperidinecarboxylate hydrochloride was obtained. Calculated molweight: 405.2250 [M+H]+. Found ISP-TOF-MS: 405.2921 [M+H]+.

Example 107: In analogy to example 1 and using the corresponding appropriate starting materials 2-({3-[amino (imino)-methyl]phenyl}amino)-N-(2-oxo-2-{[1-(phenylmethyl)-4-piperidinyl]amino}ethyl)acetamide hydrochloride was obtained. Calculated molweight: 423.2508 [M+H]+. Found ISP-TOF-MS: 423.3321 [M+H]+.

Example 108: In analogy to example 1 and using the corresponding appropriate starting materials 2-({3-[amino (imino)-methyl]phenyl}amino)-N-{1-[(cyclohexylamino)carbonyl]-2-methylpropyl}-3,4-dihydroxybutanamide hydrochloride was obtained. Calculated molweight: 434.2767 [M+H]+. Found ISP-TOF-MS: 434.3632 [M+H]+.

Example 109: In analogy to example 1 and using the corresponding appropriate starting materials 2-[({4-[amino(imino)methyl]phenyl}methyl)amino]-N-(2-{[2-(4-hydroxyphenyl)ethyl]amino}-2-oxoethyl)acetamide hydrochloride was obtained. Calculated molweight: 384.2036 [M+H]+. Found ISP-TOF-MS: 384.2610 [M+H]+.

Example 110: In analogy to example 1 and using the corresponding appropriate starting materials 2-({3-[amino (imino)-methyl]phenyl}amino)-3,4-dihydroxy-N-[2-oxo-2-(1,2,3,4-tetrahydro-1-naphthalenylamino)ethyl]butanamide hydrochloride was obtained. Calculated molweight: 440.2298 [M+H]+. Found ISP-TOF-MS: 440.2916 [M+H]+.

Example 111: In analogy to example 1 and using the corresponding appropriate starting materials 2-({3-[amino (imino)-methyl]phenyl}amino)-N-[2-(cyclohexylamino)-1-methyl-2-oxoethyl]-3,4-dihydroxybutanamide hydrochloride was obtained. Calculated molweight: 406.2454 [M+H]+. Found ISP-TOF-MS: 406.3298 [M+H]+.

Example 112: In analogy to example 1 and using the corresponding appropriate starting materials 2-[({4-[amino(imino)methyl]phenyl}methyl)amino]-N-(2-oxo-2-{[2-(2-pyridinyl)ethyl]amino}ethyl)-2- [4-(phenyloxy)phenyl]-acetamide hydrochloride was obtained. Calculated molweight: 537.2614 [M+H]+. Found ISP-TOF-MS: 537.3347 [M+H]+.

Example 113: In analogy to example 1 and using the corresponding appropriate starting materials 2-({3-[amino (imino)-methyl]phenyl}amino)-N-[2-(cyclohexylamino)-1-methyl-2-oxoethyl]-3-hydroxypropanamide hydrochloride was obtained. Calculated molweight: 376.2349 [M+H]+. Found ISP-TOF-MS: 376.2927 [M+H]+.

Example 114: In analogy to example 1 and using the corresponding appropriate starting materials 1,1-dimethylethyl 2-[({[2-({3-[amino(imino)methyl]phenyl}amino)-3,4-dihydroxybutanoyl]amino}acetyl)amino]ethylcarbamate hydrochloride was obtained. Calculated molweight: 453.2462 [M+H]+. Found ISP-TOF-MS: 453.3328 [M+H]+.

Example 115: In analogy to example 1 and using the corresponding appropriate starting materials 2-({3-[amino (imino)-methyl]phenyl}amino)-N-{2-[(diphenylmethyl)amino]-2-oxoethyl}acetamide hydrochloride was obtained. Calculated molweight: 416.2086 [M+H]+. Found ISP-TOF-MS: 416.2765 [M+H]+.

Example 116: In analogy to example 1 and using the corresponding appropriate starting materials ethyl 2-[({4-[amino(imino)methyl]phenyl}methyl)amino]-3-oxo-3-[(2-oxo-2-{[2-(2-pyridinyl)ethyl]amino}ethyl)amino]propanoate hydrochloride was obtained. Calculated molweight: 441.2250 [M+H]+. Found ISP-TOF-MS: 441.2938 [M+H]+.

Example 117: In analogy to example 1 and using the corresponding appropriate starting materials 2-[({4-[amino(imino)methyl]phenyl}methyl)amino]-N-{2-[4-(1,3-benzodioxol-5-ylmethyl)-1-piperazinyl]-2-oxoethyl}-3-hydroxypropanamide hydrochloride was obtained. Calculated molweight: 497.2512 [M+H]+. Found ISP-TOF-MS: 497.2930 [M+H]+.

Example 118: In analogy to example 1 and using the corresponding appropriate starting materials 2-[({4-[amino(imino)methyl]phenyl}methyl)amino]-N-(2-oxo-2-{[1-(phenylmethyl)-4-piperidinyl]amino}ethyl)-2-(1H-pyrazol-3-yl) acetamide hydrochloride was obtained. Calculated mol-weight: 503.2883 [M+H]+. Found ISP-TOF-MS: 503.3736 [M+H]+.

Example 119: In analogy to example 1 and using the corresponding appropriate starting materials 2-({3-[amino (imino)-methyl]phenyl}amino)-N-(2-{[2-(4-hydroxyphenyl)ethyl]amino}-2-oxoethyl)-2-(2-pyridinyl)acetamide hydrochloride was obtained. Calculated molweight: 447.2145 [M+H]+. Found ISP-TOF-MS: 447.2956 [M+H]+.

Example 120: In analogy to example 1 and using the corresponding appropriate starting materials 2-({3-[amino (imino)-methyl]phenyl}amino)-N-[2-(3,4-dihydro-2(1H)-isoquinolinyl)-2-oxoethyl]-3-methylbutanamide hydrochloride was obtained. Calculated molweight: 408.2399 [M+H]+. Found ISP-TOF-MS: 408.3045 [M+H]+.

Example 121: In analogy to example 1 and using the corresponding appropriate starting materials 2-[({4-[amino(imino)methyl]phenyl}methyl)amino]-N-{2-oxo-2-[(2-phenylethyl)amino]ethyl}propanamide hydrochloride was obtained. Calculated molweight: 382.2243 [M+H]+. Found ISP-TOF-MS: 382.2730 [M+H]+.

Example 122: In analogy to example 1 and using the corresponding appropriate starting materials 2-({3-[amino (imino)-methyl]phenyl}amino)-N-(2-{[2-(1H-indol-3-yl)ethyl]amino}-2-oxoethyl)-2-(2-pyridinyl)acetamide hydrochloride was obtained. Calculated molweight: 470.2304 [M+H]+. Found ISP-TOF-MS: 470.3156 [M+H)+.

Example 123: In analogy to example 1 and using the corresponding appropriate starting materials 2-[({4-[amino(imino)methyl]phenyl}methyl)amino]-N-[2-(3,4-dihydro-2(1H)-isoquinolinyl)-2-oxoethyl]-3-hydroxypropanamide hydrochloride was obtained. Calculated molweight: 410.2192 [M+H]+. Found ISP-TOF-MS: 410.2633 [M+H]+.

Example 124: In analogy to example 1 and using the corresponding appropriate starting materials 2-[({4-[amino(imino)methyl]phenyl}methyl)amino]-N-{2-oxo-2-[(3-pyridinylmethyl)amino]ethyl}acetamide hydrochloride was obtained. Calculated molweight: 355.1882 [M+H]+. Found ISP-TOF-MS: 355.2446 [M+H]+.

Example 125: In analogy to example 1 and using the corresponding appropriate starting materials methyl 4-{1-({3-[amino(imino)methyl]phenyl}amino)-2-oxo-2-[(2-oxo-2-{[2-(2-pyridinyl)ethyl]amino}ethyl)amino]ethyl}benzoate hydrochloride was obtained. Calculated molweight: 489.2250 [M+H]+. Found ISP-TOF-MS: 489.3128 [M+H]+.

Example 126: In analogy to example 1 and using the corresponding appropriate starting materials 2-({3-[amino (imino)-methyl]phenyl}amino)-2-cyclopropyl-N-(2-{[2-(1H-indol-3-yl)-ethyl]amino}-2-oxoethyl)acetamide hydrochloride was obtained. Calculated molweight: 433.2352 [M+H]+. Found ISP-TOF-MS: 433.2923 [M+H]+.

Example 127: In analogy to example 1 and using the corresponding appropriate starting materials 2-({3-[amino (imino)-methyl]phenyl}amino)-N-[2-({[3,4-bis(methyloxy)phenyl]-methyl}amino)-1-methyl-2-oxoethyl]-3-hydroxypropanamide hydrochloride was obtained. Calculated molweight: 444.2247 [M+H]+. Found ISP-TOF-MS: 444.2926 [M+H] +.

Example 128: In analogy to example 1 and using the corresponding appropriate starting materials 2-({3-[amino (imino)-methyl]phenyl}amino)-N-{2-[(3,3-diphenylpropyl]amino]-1-methyl-2-oxoethyl)-3,4-dihydroxybutanamide hydrochloride was obtained. Calculated molweight: 518.2767 [M+H]+. Found ISP-TOF-MS: 518.3709 [M+H]+.

Example 129: In analogy to example 1 and using the corresponding appropriate starting materials 2-[({4-[amino(imino)methyl]phenyl}methyl)amino]-N-[2-({[3,4-bis (methyloxy)phenyl]methyl}amino)-2-oxoethyl]acetamide hydrochloride was obtained. Calculated molweight: 414.2141 [M+H]+. Found ISP-TOF-MS: 414.2777 [M+H]+.

Example 130: In analogy to example 1 and using the corresponding appropriate starting materials 2-({2-[({4-[amino(imino)methyl]phenyl}methyl)amino]-3-hydroxypropanoyl}amino)-3-methyl-N-[2-(4-morpholinyl)ethyl]butanamide hydrochloride was obtained. Calculated molweight: 449.2876 [M+H]+. Found ISP-TOF-MS: 449.3324 [M+H] +.

Example 131: In analogy to example 1 and using the corresponding appropriate starting materials 2-({3-[amino (imino)-methyl]phenyl}amino)-N-{2-oxo-2-[(4-pyridinylmethyl)amino]-ethyl}acetamide hydrochloride was obtained. Calculated molweight: 341.1726 [M+H]+. Found ISP-TOF-MS: 341.2382 [M+H]+.

Example 132: In analogy to example 1 and using the corresponding appropriate starting materials 2-({3-(amino (imino)-methyl]phenyl}amino)-N-{2-[(diphenylmethyl)amino]-2-oxoethyl}-2-(2-pyridinyl)acetamide hydrochloride was obtained. Calculated molweight: 493.2352 [M+H]+. Found ISP-TOF-MS: 493.3139 [M+H]+.

Example 133: In analogy to example 1 and using the corresponding appropriate starting materials 2-({3-[amino (imino)-methyl]phenyl}amino)-N-{2-[(diphenylmethyl)amino]-2-oxo-ethyl}-3-hydroxypropanamide hydrochloride was obtained. Calculated molweight: 446.2192 [M+H]+. Found ISP-TOF-MS: 446.2799 [M+H]+.

Example 134: In analogy to example 1 and using the corresponding. appropriate starting materials 2-[({4-[amino(imino)methyl]phenyl}methyl)amino]-3-hydroxy-N-{2-oxo-2-[(4-pyridinylmethyl)amino]ethyl)propanamide hydrochloride was obtained. Calculated molweight: 385.1988 [M+H]+. Found ISP-TOF-MS: 385.2410 [M+H]+.

Example 135: In analogy to example 1 and using the corresponding appropriate starting materials 2-{[({3-[amino(imino)methyl]phenyl}amino)acetyl]amino}-N-{[3,4-bis(methyloxy)phenyl]methyl}propanamide hydrochloride was obtained. Calculated molweight: 414.2141 [M+H]+. Found ISP-TOF-MS: 414.2739 [M+H]+.

Example 136: In analogy to example 1 and using the corresponding appropriate starting materials 2-[({4-[amino(imino)methyl]phenyl}methyl)amino]-3-hydroxy-N-(2-{[2-(4-hydroxyphenyl)ethyl]amino}-2-oxoethyl)propanamide

hydrochloride was obtained. Calculated molweight: 414.2141 [M+H]+. Found ISP-TOF-MS: 414.2601 [M+H]+.

Example 137: In analogy to example 1 and using the corresponding appropriate starting materials 2-[({4-[amino(imino)methyl]phenyl}methyl)amino]-N-(2-oxo-2-{[1-(phenylmethyl)-4-piperidinyl]amino}ethyl)-2-(2-pyridinyl)-acetamide hydrochloride was obtained. Calculated molweight: 514.2930 [M+H]+. Found ISP-TOF-MS: 514.3614 [M+H]+.

Example 138: In analogy to example 1 and using the corresponding appropriate starting materials 2-[({4-[amino(imino)methyl]phenyl}methyl)amino]-N-{2-[4-(1,3-benzodioxol-5-ylmethyl)-1-piperazinyl]-2-oxoethyl}-2-(1H-pyrazol-3-yl)acetamide hydrochloride was obtained. Calculated mol-weight: 533.2625 [M+H]+. Found ISP-TOF-MS: 533.3468 [M+H]+.

Example 139: In analogy to example 1 and using the corresponding appropriate starting materials 2-({3-[amino(imino)-methyl]phenyl}amino)-N-(2-oxo-2-(4-thiomorpholinyl)ethyl]-acetamide hydrochloride was obtained. Calculated molweight: 336.1494.[M+H]+. Found ISP-TOF-MS: 336.2039 [M+H]+.

Example 140: In analogy to example 1 and using the corresponding appropriate starting materials 2-({3-[amino(imino)-methyl]phenyl}amino)-N-{2-[(3,3-diphenylpropyl)amino]-1-methyl-2-oxoethyl)-3-hydroxypropanamide hydrochloride was obtained. Calculated molweight: 488.2662 [M+H]+. Found ISP-TOF-MS: 488.3338 [M+H]+.

Example 141: In analogy to example 1 and using the corresponding appropriate starting materials ethyl 2-({3-[amino(imino)methyl]phenyl}amino)-3-({2-[(3,3-diphenylpropyl)amino]-1-methyl-2-oxoethyl}amino)-3-oxopropanoate hydrochloride was obtained. Calculated molweight: 530.2767 [M+H]+. Found ISP-TOF-MS: 530.3765 [M+H]+.

Example 142: In analogy to example 1 and using the corresponding appropriate starting materials methyl 4-[1-[({4-[amino(imino)methyl]phenyl}methyl)amino]-2-({2-[4-(1,3-benzodioxol-5-ylmethyl)-1-piperazinyl]-2-oxoethyl}amino)-2-oxoethyl]benzoate hydrochloride was obtained. Calculated molweight: 601.2775 [M+H]+. Found ISP-TOF-MS: 601.3684 [M+H]+.

Example 143: In analogy to example 1 and using the corresponding appropriate starting materials ethyl 4-{[({2-[({4-[amino(imino)methyl]phenyl}methyl)amino]-3-hydroxypropanoyl}amino)acetyl]amino}-1-piperidinecarboxylate hydrochloride was obtained. Calculated molweight: 449.2512 [M+H]+. Found ISP-TOF-MS: 449.2954 [M+H]+.

Example 144: In analogy to example 1 and using the corresponding appropriate starting materials 2-({2-[({4-[amino(imino)methyl]phenyl}methyl)amino]-3-hydroxypropanoyl)amino)-N-[2-(1H-indol-3-yl)ethyl]-3-methylbutanamide hydrochloride was obtained. Calculated molweight: 479.2771 [M+H]+. Found ISP-TOF-MS: 479.3213 [M+H]+.

Example 145: In analogy to example 1 and using the corresponding appropriate starting materials 2-[({4-[amino(imino)methyl]phenyl}methyl)amino]-N-(2-{[2-(4-morpholinyl)ethyl]amino}-2-oxoethyl)acetamide hydrochloride was obtained. Calculated molweight: 377.2301 [M+H]+. Found ISP-TOF-MS: 377.2935 [M+H]+.

Example 146: In analogy to example 1 and using the corresponding appropriate starting materials 2-({3-[amino(imino)-methyl]phenyl}amino)-N-(2-oxo-2-{[2-(2-pyridinyl)ethyl]-amino}ethyl)acetamide hydrochloride was obtained. Calculated molweight: 355.1882 [M+H]+. Found ISP-TOF-MS: 355.2469 [M+H]+.

Example 147: In analogy to example 1 and using the corresponding appropriate starting materials 1,1-dimethylethyl 2-{[({2-[({4-[amino(imino)methyl]phenyl}methyl)amino]-3-hydroxypropanoyl}amino)acetyl]amino}ethylcarbamate hydrochloride was obtained. Calculated molweight: 437.2512 [M+H]+. Found ISP-TOF-MS: 437.2948 [M+H]+.

Example 148: In analogy to example 1 and using the corresponding appropriate starting materials ethyl 2-({3-[amino(imino)methyl]phenyl}amino)-3-[(2-{[2-(4-hydroxyphenyl)ethyl]amino}-2-oxoethyl)amino]-3-oxopropanoate hydrochloride was obtained. Calculated molweight: 442.2090 [M+H]+. Found ISP-TOF-MS: 442.2951 [M+H]+.

Example 149: In analogy to example 1 and using the corresponding appropriate starting materials 2-[({4-[amino(imino)methyl]phenyl}methyl)amino]-N-{2-oxo-2-[(3-pyridinylmethyl)amino]ethyl}-2-(2-pyridinyl)acetamide hydrochloride was obtained. Calculated molweight: 432.2148 [M+H]+. Found ISP-TOF-MS: 432.2611 [M+H]+.

Example 150: In analogy to example 1 and using the corresponding appropriate starting materials 2-[({4-[amino (imino)methyl]phenyl}methyl)amino]-N-[2-({[2- (methyloxy)phenyl]methyl}amino)-2-oxoethyl]-2-(2-pyridinyl)-acetamide hydrochloride was obtained. Calculated molweight: 461.2301 [M+H]+. Found ISP-TOF-MS: 461.2944 [M+H]+.

Example 151: In analogy to example 1 and using the corresponding appropriate starting materials ethyl 4-{[({2-[({4-[amino (imino)methyl]phenyl}methyl)amino]-3,4-dihydroxybutanoyl}amino)acetyl]amino}-1-piperidinecarboxylate hydrochloride was obtained. Calculated molweight: 479.2618 [M+H]+. Found ISP-TOF-MS: 479.3297 [M+H]+.

Example 152: In analogy to example 1 and using the corresponding appropriate starting materials ethyl 4-[({[2-({3-[amino(imino)methyl]phenyl}amino)-3,4-dihydroxybutanoyl]-amino}acetyl)amino]-1-piperidinecarboxylate hydrochloride was obtained. Calculated molweight: 465.2462 [M+H]+. Found ISP-TOF-MS: 465.3338 [M+H]+.

Example 153: In analogy to example 1 and using the corresponding appropriate starting materials 2-[({4-[amino(imino)methyl]phenyl}methyl)amino]-N-{2-oxo-2-[(3-pyridinylmethyl)amino]ethyl}acetamide hydrochloride was obtained. Calculated molweight: 355.1882 [M+H]+. Found ISP-TOF-MS: 355.2333 [M+H]+.

Example 154: In analogy to example 1 and using the corresponding appropriate starting materials 2-({3-[amino(imino)-methyl]phenyl}amino)-3,4-dihydroxy-N-{2-oxo-2-[(2-phenyl-ethyl)amino]ethyl}butanamide hydrochloride was

obtained. Calculated molweight: 414.2141 [M+H]+. Found ISP-TOF-MS: 414.2975 [M+H]+.

Example 155: In analogy to example 1 and using the corresponding appropriate starting materials 2-[({4-[amino(imino)methyl]phenyl}methyl)amino]-3-hydroxy-N-{2-oxo-2-[(2-phenylethyl)amino]ethyl}propanamide hydrochloride was obtained. Calculated molweight: 398.2192 [M+H]+. Found ISP-TOF-MS: 398.2621 [M+H]+.

Example 156: In analogy to example 1 and using the corresponding appropriate starting materials 2-[({4-[amino(imino)methyl]phenyl}methyl)amino]-N-(2-oxo-2-{(2-(2-pyridinyl)ethyl]amino}ethyl)-2-(2-pyridinyl)acetamide hydrochloride was obtained. Calculated molweight: 446.2304 [M+H]+. Found ISP-TOF-MS: 446.2879 [M+H]+.

Example 157: In analogy to example 1 and using the corresponding appropriate starting materials 2-({3-[amino(imino)-methyl]phenyl}amino)-N-(2-oxo-2-{[1-(phenylmethyl)-4-piperidinyl]amino}ethyl)-2-(2-pyridinyl)acetamide hydrochloride was obtained. Calculated molweight: 500.2774 [M+H]+. Found ISP-TOF-MS: 500.3613 [M+H]+.

Example 158: In analogy to example 1 and using the corresponding appropriate starting materials 2-({3-[amino(imino)-methyl]phenyl}amino)-N-[2-({2-[3,4-bis(methyloxy)phenyl]-ethyl}amino)-2-oxoethyl]acetamide hydrochloride was obtained. Calculated molweight: 414.2141 [M+H]+. Found ISP-TOF-MS: 414.2856 [M+H)+.

Example 159: In analogy to example 1 and using the corresponding appropriate starting materials 2-[({4-[amino(imino)methyl]phenyl}methyl)amino]-3-hydroxy-N-[2-oxo-2-(1,2,3,4-tetrahydro-1-naphthalenylamino)ethyl]propanamide hydrochloride was obtained. Calculated molweight: 424.2349 [M+H]+. Found ISP-TOF-MS: 424.2767 [M+H]+.

Example 160: In analogy to example 1 and using the corresponding appropriate starting materials 2-[({4-[amino(imino)methyl]phenyl}methyl)amino]-N-{2-oxo-2-[(4-pyridinylmethyl)amino] ethyl}-2- [4-(phenyloxy)phenyl]-acetamide hydrochloride was obtained. Calculated molweight: 523.2458 [M+H]+. Found ISP-TOF-MS: 523.3143 [M+H]+.

Example 161: In analogy to example 1 and using the corresponding appropriate starting materials 2-({3-[amino(imino)-methyl]phenyl}amino)-N-[2-oxo-2-(1,2,3,4-tetrahydro-1-naphthalenylamino)ethyl]acetamide hydrochloride was obtained. Calculated molweight: 380.2086 [M+H]+. Found ISP-TOF-MS: 380.2715 [M+H]+.

Example 162: In analogy to example 1 and using the corresponding appropriate starting materials 2-({3-[amino(imino)-methyl]phenyl}amino)-N-[2-({[3,4-bis(methyloxy)phenyl]-methyl}amino)-2-oxoethyl]-3,4-dihydroxybutanamide hydrochloride was obtained. Calculated molweight: 460.2196 [M+H]+. Found ISP-TOF-MS: 460.3059 [M+H]+.

Example 163: In analogy to example 1 and using the corresponding appropriate starting materials 2-[({4-[amino(imino)methyl]phenyl}methyl)amino]-3-hydroxy-N-(2-{[2-(4-morpholinyl)ethyl]amino}-2-oxoethyl)propanamide hydrochloride was obtained. Calculated molweight: 407.2407 [M+H]+. Found ISP-TOF-MS: 407.2810 [M+H]+.

Example 164: In analogy to example 1 and using the corresponding appropriate starting materials 2-({[({4-[amino(imino)methyl]phenyl}methyl)amino]acetyl}amino)-3-methyl-N-(phenylmethyl)butanamide hydrochloride was obtained. Calculated molweight: 396.2399 [M+H]+. Found ISP-TOF-MS: 396.3039 [M+H]+.

Example 165: In analogy to example 1 and using the corresponding appropriate starting materials 2-[({4-[amino(imino)methyl)phenyl}methyl)amino]-N- (2-{[2-(4-hydroxyphenyl)ethyl]amino}-2-oxoethyl)propanamide hydrochloride was obtained. Calculated molweight: 398.2192 [M+H]+. Found ISP-TOF-MS: 398.2696 [M+H]+.

Example 166: In analogy to example 1 and using the corresponding appropriate starting materials 2-[({4-[amino(imino)methyl]phenyl}methyl)amino]-3-hydroxy-N-[2-oxo-2-(4-thiomorpholinyl)ethyl]propanamide hydrochloride was obtained. Calculated molweight: 380.1756 (M+H]+. Found ISP-TOF-MS: 380.2151 [M+H]+.

Example 167: In analogy to example 1 and using the corresponding appropriate starting materials 2-({3-[amino(imino)-methyl]phenyl}amino)-3,4-dihydroxy-N-(2-methyl-1-{[(phenylmethyl)amino)carbonyl}propyl)butanamide hydrochloride was obtained. Calculated molweight: 442.2454 [M+H]+. Found ISP-TOF-MS: 442.3424 [M+H]+.

Example 168: In analogy to example 1 and using the corresponding appropriate starting materials 2-[({4-[amino(imino)methyl]phenyl}methyl)amino]-N-(2-oxo-2-[(4-pyridinylmethyl)amino]ethyl}acetamide hydrochloride was obtained. Calculated molweight: 355.1882 [M+H]+. Found ISP-TOF-MS: 355.2399 [M+H]+.

Example 169: In analogy to example 1 and using the corresponding appropriate starting materials 2-{[2-({3-[amino(imino)methyl]phenyl}amino)-3-hydroxypropanoyl]amino}-3-methyl-N- [2- (4-morpholinyl) ethyl]butanamide hydrochloride was obtained. Calculated molweight: 435.2720 [M+H]+. Found ISP-TOF-MS: 435.3332 [M+H]+.

Example 170: In analogy to example 1 and using the corresponding appropriate starting materials 2-({3-[amino(imino)-methyl]phenyl}amino)-N-(2-oxo-2-{[1-(phenylmethyl)-4-piperidinyl]amino}ethyl)-2-(1H-pyrazol-3-yl)acetamide hydrochloride was obtained. Calculated molweight: 489.2726 (M+H]+. Found ISP-TOF-MS: 489.3648 [M+H]+.

Example 171: In analogy to example 1 and using the corresponding appropriate starting materials 2-[({4-[amino(imino)methyl]phenyl}methyl)amino]-N-[2-({2-[3,4-bis(methyloxy)phenyl]ethyl}amino)-2-oxoethyl]-2-(2-pyridinyl) acetamide hydrochloride was obtained. Calculated mol-weight: 505.2563 [M+H]+. Found ISP-TOF-MS: 505.3239 [M+H]+.

Example 172: In analogy to example 1 and using the corresponding appropriate starting materials ethyl 2-({3-[amino(imino)methyl]phenyl}amino)-3-({2-[(diphenylmethyl)-amino]-2-oxoethyl}amino)-3-oxopropanoate hydrochloride was obtained. Calculated molweight: 488.2298 [M+H]+. Found ISP-TOF-MS: 488.3264 [M+H]+.

Example 173: In analogy to example 1 and using the corresponding appropriate starting materials 2-({3-[amino(imino)-methyl]phenyl}amino)-N-[2-({[3,4-bis(methyloxy)phenyl]-methyl}amino)-1-methyl-2-oxoethyl]-3,4-dihydroxybutanamide hydrochloride was obtained. Calculated molweight: 474.2353 [M+H]+. Found ISP-TOF-MS: 474.3279 [M+H]+.

Example 174: In analogy to example 1 and using the corresponding appropriate starting materials 2-[({4-[amino(imino)methyl]phenyl}methyl)amino]-N-(2-{[2-(1H-indol-3-yl)ethyl]amino}-2-oxoethyl)-2-(2-pyridinyl)acetamide hydrochloride was obtained. Calculated molweight: 484.2461 [M+H]+. Found ISP-TOF-MS: 484.3020 [M+H]+.

Example 175: In analogy to example 1 and using the corresponding appropriate starting materials 2-({3-(amino (imino)-methyl]phenyl}amino)-3,4-dihydroxy-N-{2-oxo-2-[4-(2-pyrimidinyl)-1-piperazinyl]ethyl}butanamide hydrochloride was obtained. Calculated molweight: 457.2312 [M+H]+. Found ISP-TOF-MS: 457.3193 [M+H]+.

Example 176: In analogy to example 1 and using the corresponding appropriate starting materials 2-{[({3-[amino(imino)methyl]phenyl}amino)(1H-pyrazol-3-yl)acetyl]-amino}-N-[2-(1H-indol-3-yl)ethyl]-3-methylbutanamide hydrochloride was obtained. Calculated molweight: 501.2726 [M+H]+. Found ISP-TOF-MS: 501.3640 [M+H]+.

Example 177: In analogy to example 1 and using the corresponding appropriate starting materials methyl 4-(1-[({4-[amino(imino)methyl]phenyl}methyl)amino]-2-{[2-({[3,4-bis(methyloxy)phenyl]methyl)amino}-1-methyl-2-oxoethyl]-amino}-2-oxoethyl)benzoate hydrochloride was obtained. Calculated molweight: 562.2666 [M+H]+. Found ISP-TOF-MS: 562.3569 [M+H]+.

Example 178: In analogy to example 1 and using the corresponding appropriate starting materials 2-[({4-[amino(imino)methyl]phenyl}methyl)amino]-N-(2-oxo-2-{[1-(phenylmethyl) -4-piperidinyl]amino}ethyl)propanamide hydrochloride was obtained. Calculated molweight: 451.2821 [M+H]+. Found ISP-TOF-MS: 451.3436 [M+H]+.

Example 179: In analogy to example 1 and using the corresponding appropriate starting materials ethyl 4-[({[({3-[amino(imino)methyl]phenyl}amino) (2-pyridinyl)acetyl]amino}-acetyl)amino]-1-piperidinecarboxylate hydrochloride was obtained. Calculated molweight: 482.2516 [M+H]+. Found ISP-TOF-MS: 482.3383 [M+H]+.

Example 180: In analogy to example 1 and using the corresponding appropriate starting materials ethyl 2-({3-[amino(imino)methyl]phenyl}amino)-3-{(2-(3,4-dihydro-2(1H)-isoquinolinyl)-2-oxoethyl]amino}-3-oxopropanoate hydrochloride was obtained. Calculated molweight: 438.2141 [M+H]+. Found ISP-TOF-MS: 438.2997 [M+H]+.

Example 181: In analogy to example 1 and using the corresponding appropriate starting materials ethyl 2-({3-[amino(imino)methyl]phenyl}amino)-3-[(2-{4-[2-(methyloxy)-phenyl]-1-piperazinyl}-2-oxoethyl) amino]-3-oxopropanoate hydrochloride was obtained. Calculated molweight: 497.2512 [M+H]+. Found ISP-TOF-MS: 497.3395 [M+H]+.

Example 182: In analogy to example 1 and using the corresponding appropriate starting materials 2-[({4-[amino(imino)methyl]phenyl}methyl)amino]-N-{2-oxo-2-[ (4-pyridinylmethyl)amino]ethyl}acetamide hydrochloride was obtained. Calculated molweight: 355.1882 [M+H]+. Found ISP-TOF-MS: 355.2340 [M+H]+.

Example 183: In analogy to example 1 and using the corresponding appropriate starting materials 2-[({4-[amino (imino)methyl]phenyl}methyl)amino]-2-[5- (methyloxy)-1H-indol-3-yl]-N-{2-oxo-2-[(4-pyridinylmethyl)amino]ethyl}-acetamide hydrochloride was obtained. Calculated molweight: 500.2410 [M+H]+. Found ISP-TOF-MS: 500.3257 [M+H]+.

Example 184: In analogy to example 1 and using the corresponding appropriate starting materials ethyl 4-{[({2-[({4-[amino(imino)methyl]phenyl}methyl)amino]propanoyl}amino)acetyl]amino}-1-piperidinecarboxylate hydrochloride was obtained. Calculated molweight: 433.2563 [M+H]+. Found ISP-TOF-MS: 433.3073 [M+H]+.

Example 185: In analogy to example 1 and using the corresponding appropriate starting materials 2-[({4-[amino(imino)methyl]phenyl}methyl)amino]-3,4-dihydroxy-N-{2-oxo-2-[(4-pyridinylmethyl)amino]ethyl}butanamide hydrochloride was obtained. Calculated molweight: 415.2094 [M+H]+. Found ISP-TOF-MS: 415.2756 [M+H]+.

Example 186: In analogy to example 1 and using the corresponding appropriate starting materials 2-({3-[amino (imino)-methyl]phenyl}amino)-N-{2-[(3,3-diphenylpropyl)amino]-2-oxo-ethyl}acetamide hydrochloride was obtained. Calculated molweight: 444.2399 [M+H]+. Found ISP-TOF-MS: 444.3123 [M+H]+.

Example 187: In analogy to example 1 and using the corresponding appropriate starting materials 2-[({4-[amino(imino)methyl]phenyl}methyl)amino]-N-[2-(cyclohexylamino)-2-oxoethyl]propanamide hydrochloride was obtained. Calculated molweight: 360.2399 [M+H]+. Found ISP-TOF-MS: 360.2958 [M+H]+.

Example 188: In analogy to example 1 and using the corresponding appropriate starting materials 2-({3-[amino (imino)-methyl]phenyl}amino)-N-[1-({[2-(1H-indol-3-yl)ethyl]amino}-carbonyl)-2-methylpropyl]-3-methylbutanamide hydrochloride was obtained. Calculated molweight: 477.2978 [M+H]+. Found ISP-TOF-MS: 477.3790 [M+H]+.

Example 189: In analogy to example 1 and using the corresponding appropriate starting materials 2-[({4-[amino(imino)methyl]phenyl}methyl)amino]-2-[5-(methyloxy)-1H-indol-3-yl]-N-(2-oxo-2-{[2-(2-pyridinyl)ethyl]amino}-ethyl) acetamide hydrochloride was obtained. Calculated molweight: 514.2567 [M+H]+. Found ISP-TOF-MS: 514.3214 [M+H]+.

Example 190: In analogy to example 1 and using the corresponding appropriate starting materials 2-({3-[amino (imino)-methyl]phenyl}amino)-N-(2-{[2-(4-morpholinyl)ethyl]amino}-2-oxoethyl)-2-(1H-pyrrol-2-yl)acetamide hydrochloride was obtained. Calculated molweight: 428.2410 [M+H]+. Found ISP-TOF-MS: 428.3253 [M+H]+.

Example 191: In analogy to example 1 and using the corresponding appropriate starting materials 2-[({4-[amino(imino)methyl]phenyl}methyl)amino]-N-[2-(3,4-dihydro-2(1H)-isoquinolinyl)-2-oxoethyl]acetamide hydrochloride

was obtained. Calculated molweight: 380.2086 [M+H]+. Found ISP-TOF-MS: 380.2636 [M+H]+.

Example 192: In analogy to example 1 and using the corresponding appropriate starting materials ethyl 2-({3-[amino (imino)methyl]phenyl}amino) -3-oxo-3- ((2-oxo-2- [4-(2-pyrimidinyl)-1-piperazinyl]ethyl}amino)propanoate hydrochloride was obtained. Calculated molweight: 469.2312 [M+H]+. Found ISP-TOP-MS: 469.3198 [M+H]+.

Example 193: In analogy to example 1 and using the corresponding appropriate starting materials 2-[({4-[amino(imino)methyl]phenyl}methyl)amino]-N-[2-({[3,4-bis-(methyloxy)phenyl]methyl}amino)-2-oxoethyl]-3-hydroxypropanamide. hydrochloride was obtained. Calculated molweight: 444.2247 [M+H]+. Found ISP-TOF-MS: 444.2696 [M+H]+.

Example 194: In analogy to example 1 and using the corresponding appropriate starting materials 2-[({4-[amino (imino) methyl]phenyl}methyl)amino]-N-(2-{[2- (1H-indol-3-yl)ethyl]amino}-2-oxoethyl)-3-methylbutanamide hydrochloride was obtained. Calculated molweight: 449.2665 [M+H]+. Found ISP-TOF-MS: 449.3250 [M+H]+.

Example 195: In analogy to example 1 and using the corresponding appropriate starting materials 2-({[({4-[amino(imino)methyl]phenyl}methyl)amino]acetyl}amino)-3-methyl-N- [2- (4-morpholinyl)ethyl]butanamide hydrochloride was obtained. Calculated molweight: 419.2771 [M+H]+. Found ISP-TOF-MS: 419.3429 [M+H]+.

Example 196: In analogy to example 1 and using the corresponding appropriate starting materials 2-{[({3-[amino(imino)methyl]phenyl}amino)acetyl]amino}-3-methyl-N-[2-(4-morpholinyl)ethyl]butanamide hydrochloride was obtained. Calculated molweight: 405.2614 [M+H]+. Found ISP-TOF-MS: 405.3295 [M+H]+.

Example 197: In analogy to example 1 and using the corresponding appropriate starting materials ethyl 2-({3-[amino(imino)methyl]phenyl}amino)-3-({2-[4-(1,3-benzodioxol-5-ylmethyl)-1-piperazinyl] -2-oxoethyl}amino) -3-oxopropanoate hydrochloride was obtained. Calculated molweight: 525.2462 [M+H]+. Found ISP-TOF-MS: 525.3328 [M+H]+.

Example 198: In analogy to example 1 and using the corresponding appropriate starting materials ethyl 2-({3-[amino(imino)methyl)phenyl}amino)-3-oxo-3-{[2-oxo-2-(4-thiomorpholinyl)ethyl]amino}propanoate hydrochloride was obtained. Calculated molweight: 408.1706 [M+H]+. Found ISP-TOF-MS: 408.2537 [M+H]+.

Example 199: In analogy to example 1 and using the corresponding appropriate starting materials 2-[({4-[amino(imino)methyl]phenyl}methyl)amino]-2-(1,3-benzodioxol-5-yl)-N-[2-({[3,4-bis(methyloxy)phenyl]methyl}amino)-2-oxo-ethyl]acetamide hydrochloride was obtained. Calculated molweight: 534.2353 [M+H]+. Found ISP-TOF-MS: 534.2881 [M+HJ+.

Example 200: In analogy to example 1 and using the corresponding appropriate starting materials 2-{[({3-[amino(imino)methyl]phenyl}amino)acetyl]amino}-N-cyclohexyl-3-methylbutanamide hydrochloride was obtained. Calculated molweight: 374.2556 [M+H]+. Found ISP-TOF-MS: 374.3201 [M+H]+.

Example 201: In analogy to example 1 and using the corresponding appropriate starting materials 2-({3-[amino (imino)-methyl]phenyl}amino)-N-(2-oxo-2-{[2-(2-pyridinyl)ethyl]-amino}ethyl)acetamide hydrochloride was obtained. Calculated molweight: 355.1882 [M+H]+. Found ISP-TOF-MS: 355.2497 [M+H]+.

Example 202: In analogy to example 1 and using the corresponding appropriate starting materials 2-{[({3-[amino(imino)methyl]phenyl}amino)acetyl]amino}-N-(3,3-diphenylpropyl)propanamide hydrochloride was obtained. Calculated molweight: 458.2556 [M+H]+. Found ISP-TOF-MS: 458.3160 [M+H]+.

Example 203: In analogy to example 1 and using the corresponding appropriate starting materials 2-({3-[amino (imino)-methyl]phenyl}amino)-2-cyclopropyl-N-[2-oxo-2-(4-thiomorpholinyl)ethyl]acetamide hydrochloride was obtained. Calculated molweight: 376.1807 [M+H]+. Found ISP-TOF-MS: 376.2360 [M+H]+.

Example 204: In analogy to example 1 and using the corresponding appropriate starting materials 2-[({4-[amino(imino)methyl]phenyl}methyl)amino]-2-[5-(methyloxy)-1H-indol-3-yl]-N-{2-oxo-2-[(3-pyridinylmethyl)amino]ethyl}-acetamide hydrochloride was obtained. Calculated molweight: 500.2410 [M+H]+. Found ISP-TOF-MS: 500.2811 [M+H]+.

Example 205: In analogy to example 1 and using the corresponding appropriate starting materials 2-[({4-[amino(imino)methyl]phenyl}methyl)amino]-N-(2-{[2-(1H-indol-3-yl)ethyl]amino}-2-oxoethyl)-2-(1H-pyrazol-3-yl)acetamide hydrochloride was obtained. Calculated molweight: 473.2413 [M+H]+. Found ISP-TOF-MS: 473.3060 [M+H]+.

Example 206: In analogy to example 1 and using the corresponding appropriate starting materials 2-({3-[amino (imino)-methyl]phenyl}amino)-N-(2-{[2-(4-hydroxyphenyl)ethyl]amino}-2-oxoethyl)-2-(1H-pyrazol-3-yl)acetamide hydrochloride was obtained. Calculated molweight: 436.2097 [M+H]+. Found ISP-TOF-MS: 436.2916 [M+H]+.

Example 207: In analogy to example 1 and using the corresponding appropriate starting materials 2-({3-[amino (imino)-methyl]phenyl}amino)-2-cyclopropyl-N-[2-(3,4-dihydro-2(1H)-isoquinolinyl)-2-oxoethyl]acetamide hydrochloride was obtained. Calculated molweight: 406.2243 [M+H]+. Found ISP-TOF-MS: 406.2846 [M+H]+.

Example 208: In analogy to example 1 and using the corresponding appropriate starting materials 2-({3-[amino (imino)-methyl]phenyl}amino)-N-(2-oxo-2-[(3-pyridinylmethyl)amino]-ethyl}acetamide hydrochloride was obtained. Calculated molweight: 341.1726 [M+H]+. Found ISP-TOF-MS: 341.2365 [M+H]+.

Example 209: In analogy to example 1 and using the corresponding appropriate starting materials 2-({3-[amino (imino)-methyl]phenyl}amino)-N-[2-(cyclohexylamino)-2-oxoethyl]-2-(2-pyridinyl)acetamide hydrochloride was obtained. Calculated molweight: 409.2352 [M+H)+. Found ISP-TOF-MS: 409.3039.[M+H]+.

Example 210: In analogy to example 1 and using the corresponding appropriate starting materials 2-[({4-[amino(imino)methyl)phenyl}methyl)amino]-N-{2-[(3,3-diphenylpropyl)amino]-2-oxoethyl}-3-hydroxypropanamide hydrochloride was obtained. Calculated molweight: 488.2662 [M+H]+. Found ISP-TOF-MS: 488.3194 [M+H]+.

Example 211: In analogy to example 1 and using the corresponding appropriate starting materials 1,1-dimethylethyl 2-{[((2-[({4-[amino(imino)methyl]phenyl}methyl)amino]propanoyl}amino)acetyl]amino}ethylcarbamate hydrochloride was obtained. Calculated molweight: 421.2563 [M+H]+. Found ISP-TOF-MS: 421.3079 [M+H]+.

Example 212: In analogy to example 1 and using the corresponding appropriate starting materials ethyl 2-({3-[amino(imino)methyl]phenyl}amino)-3-{[2-({[2-(methyloxy)-phenyl]methyl}amino)-2-oxoethyl]amino}-3-oxopropanoate hydrochloride was obtained. Calculated molweight: 442.2090 [M+H]+. Found ISP-TOF-MS: 442.2938 [M+H]+.

Example 213: In analogy to example 1 and using the corresponding appropriate starting materials 2-[({4-[amino(imino)methyl]phenyl}methyl)amino]-N-[2-(3,4-dihydro-2(1H)-isoquinolinyl)-2-oxoethyl]propanamide hydrochloride was obtained. Calculated molweight: 394.2243 [M+H]+. Found ISP-TOF-MS: 394.2723 [M+H]+.

Example 214: In analogy to example 1 and using the corresponding appropriate starting materials 2-{[({3-[amino(imino)methyl]phenyl}amino)acetyl]amino]-N-(1,3-benzodioxol-5-ylmethyl)-3-methylbutanamide hydrochloride was obtained. Calculated molweight: 426.2141 [M+H]+. Found ISP-TOF-MS: 426.2867 [M+H]+.

Example 215: In analogy to example 1 and using the corresponding. appropriate starting materials 2-[({4-[amino(imino)methyl]phenyl}methyl)amino]-N-(2-{[2-(4-hydroxyphenyl)ethyl]amino}-2-oxoethyl)-2-(1H-pyrazol-3-yl)-acetamide hydrochloride was obtained. Calculated molweight: 450.2254 [M+H]+. Found ISP-TOF-MS: 450.2942 [M+H]+.

Example 216: In analogy to example 1 and using the corresponding appropriate starting materials ethyl 2-({3-[amino(imino)methyl]phenyl}amino)-3-oxo-3-[(2-oxo-2-{[2-(2-pyridinyl)ethyl]amino}ethyl)amino]propanoate hydrochloride was obtained. Calculated molweight: 427.2094 [M+H]+. Found ISP-TOF-MS: 427.2929 [M+H]+.

Example 217: In analogy to example 1 and using the corresponding appropriate starting materials methyl 4-(1-({3-[amino(imino)methyl]phenyl}amino)-2-{[2-methyl-1-({[2- (2-pyridinyl)ethyl]amino}carbonyl)propyl]amino}-2-oxoethyl)-benzoate hydrochloride was obtained. Calculated molweight: 531.2720 [M+H]+. Found ISP-TOF-MS: 531.3662 [M+H]+.

Example 218: In analogy to example 1 and using the corresponding appropriate starting materials ethyl 2-({3-[amino(imino)methyl]phenyl}amino)-3-oxo-3-({2-oxo-2-[(phenylmethyl)amino]ethyl}amino)propanoate hydrochloride was obtained. Calculated molweight: 412.1985 [M+H]+. Found ISP-TOF-MS: 412.2804 [M+H]+.

Example 219: In analogy to example 1 and using the corresponding appropriate starting materials 2-[({4-[amino(imino)methyl]phenyl}methyl)amino]-N-{2-oxo-2-[(3-pyridinylmethyl)amino]ethyl}propanamide hydrochloride was obtained. Calculated molweight: 369.2039 [M+H]+. Found ISP-TOF-MS: 369.2506 [M+H]+.

Example 220: In analogy to example 1 and using the corresponding appropriate starting materials 2-[({4-[amino(imino)methyl]phenyl}methyl)amino]-N-(2-oxo-2-{[1-(phenylmethyl)-4-piperidinyl]amino}ethyl)acetamide hydrochloride. was obtained. Calculated molweight: 437.2665 [M+H]+. Found ISP-TOF-MS: 437.2982 [M+H]+.

Example 221: In analogy to example 1 and using the corresponding appropriate starting materials 2-({3-[amino (imino)-methyl]phenyl}amino)-N-{2-oxo-2-[(4-pyridinylmethyl)amino]-ethyl}-2-(2-pyridinyl)acetamide hydrochloride was obtained. Calculated molweight: 418.1991 [M+H]+. Found ISP-TOF-MS: 418.2786 [M+H]+.

Example 222: In analogy to example 1 and using the corresponding appropriate starting materials 2-({3-[amino (imino)-methyl]phenyl}amino)-N-[2-({2-[3,4-bis(methyloxy)phenyl]-ethyl}amino)-2-oxoethyl]-2-(2-pyridinyl)acetamide hydrochloride was obtained. Calculated molweight: 491.2407 [M+H]+. Found ISP-TOF-MS: 491.3170 [M+H]+.

Example 223: In analogy to example 1 and using the corresponding appropriate starting materials 2-[({4-[amino(imino)methyl]phenyl}methyl)amino]-N-{2-[4-(1,3-benzodioxol-5-ylmethyl)-1-piperazinyl]-2-oxoethyl}acetamide hydrochloride was obtained. Calculated molweight: 467.2407 [M+H]+. Found ISP-TOF-MS: 467.2710 [M+H]+.

Example 224: In analogy to example 1 and using the corresponding appropriate starting materials 2-({3-[amino (imino)-methyl]phenyl}amino)-N-(2-oxo-2-[4-(2-pyrimidinyl)-1-piperazinyl]ethyl}acetamide hydrochloride was obtained. Calculated molweight: 397.2100 [M+H]+. Found ISP-TOF-MS: 397.2755 [M+H]+.

Example 225: In analogy to example 1 and using the corresponding appropriate starting materials 2-[({4-[amino(imino)methyl]phenyl}methyl)amino]-2-(1,3-benzodioxol-5-yl)-N-{2-oxo-2-[(4-pyridinylmethyl)amino]ethyl}acetamide hydrochloride was obtained. Calculated molweight: 475.2094 [M+H]+. Found ISP-TOF-MS: 475.2640 [M+H]+.

Example 226: General procedure for the synthesis of isonitriles of Formula (IV). 25 Mmol of the corresponding amine of the Formula VIII and 25 mmol of the corresponding isonitrile of the Formula (VII) are mixed at room temperature. If no solution is formed 2 ml of methanol are added and the reaction mixture is stirred for 1 day. The reaction mixture is then suspended in 50 ml diethylether, filtered off and the solid material is washed with diethylether. Yields are between 5 and 99%.

Example 227: 2.7 g Picolylamine and 2.48 g isocyanoacetic acid methylester give according to example 226 3.98 g (91% yield) pale yellow powder of N-(4-pyridylmethyl)-2-isocyano-acetamide.

Example 228: General procedure for the synthesis of N-4-cyanobenzyl amino acids of Formula (V). 1 Mol of the corresponding amino acid is added to 500 ml of a 2N aqueous sodium hydroxide solution. The suspension is allowed to stir over 20 minutes. 1 Mol of 4-cyanobenzaldehyde are added under stirring at a temperature below 5 degrees Celsius. 0.33 Mol sodium cyanoborohydride is added in portions during a period of 30 minutes and the reaction mixture is allowed to stir over 30 minutes. An additional portion of 0.5 Mol of 4-cyanobenzaldehyde and 0.16 Mol sodium cyanoborohydride are added under cooling. After 2 hours stirring at room temperature the reaction solution is extracted with diethylether. The aqueous solution is made acidic with 2N hydrochloric acid. The product precipitates and is filtered off.

Example 229: According to the example 228 the following products were obtained:

N-(4-cyanobenzyl)alanine as a colourless powder, 36% yield, ISP-MS: 205.10 [M+H]+.
N-(4-cyanobenzyl)valine as a colourless powder, 58% yield, ISP-MS: 233.10 [M+H]+.
N-(4-cyanobenzyl)glycine as a colourless powder, 26% yield, ISP-MS: 191.00 [M+H]+.
N-(4-cyanobenzyl)serine as a colourless powder, 46% yield, ISP-MS: 221.10 [M+H]+.

Example 230: General procedure for the synthesis of N-3-cyanophenyl amino acids of Formula (V). 0.1 Mol of 3-aminobenzonitrile is dissolved in ethanol and 0.15 of the corresponding alpha-keto acid is added. 0.1 Mol acetic acid is added under stirring at room temperature. After 5 minutes 0.3 Mol of sodium cyanoborohydride is added in portions. The solvent is evaporated after 3 hours and the remainder is suspended in water and solid sodium hydroxide is added until the pH of the solution is 12. The aqueous solution is extracted three times with dichloromethane and acidified with hydrochloric acid to a pH of 2 whereby the product precipitates. The product is filtered off. In case that the product does not precipitate from the solution the aqueous phase is extracted three times with acetic acid ethyl ester, the organic phases are dried over sodium sulfate. After evaporating the solvent, acetic acid ethyl ester is added again and the solvent is evaporated again. This procedure is repeated for two times, yielding the product as a colourless powder.

Example 231: According to example 230 the following products were obtained:

N-(3-cyanophenyl)alanine as a colourless powder, 60% yield, ISP-MS: 191.00 [M+H]+.
N-(3-cyanophenyl)glycine as a colourless powder, 25% yield, ISP-MS: 177.00 [M+H]+.
N-(3-cyanophenyl)serine as a colourless powder, 6% yield, ISP-MS: 207.00 [M+H]+.

Example 232: 1 Mol 2-bromo-3-methyl-butyric acid was dissolved in methanol and at 0 degrees Celsius a 1N methanolic solution of sodium hydroxide is added until a pH of. 9 to 10 is reached. The solution is allowed to stir for an additional 5 minutes and removing than the solvent under reduced pressure until a colourless solid is obtained. 6.5 Mol of 3-aminobenzonitrile is added and the mixture is melted at 100 degrees Celsius for 3.5 hours. To the reaction product a 0.1 N aqueous sodium hydroxide solution is added and the resulting solution is stirred for 5 minutes. This emulsion is extracted with diethylether three times to remove the excess of amine. The aqueous phase is acidified with hydrochloric acid whereby the product precipitates as a colourless solid that is filtered off. Yield 70%. ISP-MS: 219.00 [M+H]+.

Example 233: 453 mg N-(3-cyanophenyl)alanine is dissolved in 20 ml of tetrahydrofurane, 308 mg carbonyldiimidazole and 408 mg 2-aminoacetyl-1,2,34-tetrahydroisochilonline are added and the suspension is stirred for 3 hours at room temperature. After addition of 100 ml water the reaction mixture is extracted three times with ethylacetate. The combined organic phases are dried and the solvent is evaporated. The remainder is crystallised from ethylacetate whereby 390 mg (49% yield) of 2-[(3-cyanophenyl)amino)]-N-[2-(3,4-dihydro-2(1H)-isoquinolinyl)-2-oxoethyl]propanamide is obtained as a colourless solid. ISP-MS: 363.10 [M+H]+.

Example 234: General for the preparation of azidomethyl benzonitriles. To a stirred 0.5 M solution of the required bromomethyl benzonitrile in dimethylformamide was added sodium azide (1.05 equiv.) under nitrogen at room temperature. The resulting reaction was followed by HPLC. After 24 hours the reaction is normally complete, and so the reaction is then evaporated down under reduced pressure, to give a crude mixture. Diethyl ether is added, the resulting mixture filtered and the solid washed twice with diethyl ether. The filtrate and washings are then concentrated down, in vacuo, to give an almost pure product. This was normally used directly for the next stage and then purified.

Example 235: According to the general procedure described in example 235 the following compounds were made:

3-Azidomethyl benzonitrile as a light brown oil (still contains small quantity of DMF) - not distilled. $R_f$ 0.30 (25% ethyl acetate in n-heptane); 1H NMR ($CDCl_3$; 400 MHz) 4.43 (2H, s, CH2), 7.64-7.49 (4H, m, Ar-H); 13C NMR ($CDCl_3$; 100 MHz) 53.518 (CH2), 112.80 (C), 118.183 (C), 129.53 (CH), 131.189 (CH), 131.656 (CH), 132.122 (CH), 136.985 (C). Mass Spectrum (CI) mz: Found $(M + H)^+$, 159. Calculated for $C_8H_6N_4$, $(M + H)^+$, 159.

4-Azidomethyl benzonitrile as a colourless oil, b.pt 85-90°C at 0.075 mmHg. $R_f$ 0.30 (25% ethyl acetate in n-heptane); 1H NMR ($CDCl_3$; 400 MHz) 4.46 (2H, s, CH2), 7.44 (2H, d, J 8.0, 2 x $H_m$), 7.67 (2H, d, J 8.0, 2 x $H_o$); 13C NMR ($CDCl_3$; 100 MHz) 53.839 (CH2), 111.936 (C), 118.275 (C), 128.352 (2 x CH), 132.428 (2 x CH), 140.663 (C); Mass Spectrum (CI) mz: Found (M + H), 159. Calculated for $C_8H_6N_4$, $(M + H)^+$, 159.

Example 236: General procedure for the preparation of aminomethyl benzonitriles. A solution of 1,2-bis(diphenylphosphino)ethane (0.49 equiv.) in dry THF (minimal volume to dissolve) is added slowly to a stirred solution of required azidomethyl benzonitrile (1 equiv) in dry THF (Fluka - puriss, 0.5 M solution) under $N_2$ at room temperature. Evolution of a gas (nitrogen) is observed and the reaction followed by TLC. After the TLC showed there to be no starting material present, distilled water (2.1 equiv.) is added. The reaction is then allowed to stir at room temperature over night, and then the mixture is evaporated down, *in vacuo.* Acetonitrile is added to the mixture, the solid filtered and the solid washed with twice with acetonitrile. The resulting filtrate and washings are evaporated down, *in vacuo,* and the crude product purified by distillation.

Example 237: 3-Aminomethyl benzonitrile was prepared according to example 236. The product was purified by distillation to give the product (70-85 % over two steps) as a colourless oil, b.p. = 68-71°C at 0.05 mm Hg; $R_f$ = 0.37 (9:0.9:0.1 Dichloromethane:methanol:ammonia); 1H NMR ($CDCl_3$; 400 MHz) 1.57 (2H, br s, NH2), 3.927 (2H, s, CH2), 7.64-7.41 (4H, m, Ar-H); 13C NMR ($CDCl_3$; 100 MHz) 45.298 (CH2), 112.12 (C), 118.68 (C), 128.96 (CH), 130.17 (CH), 130.39 (CH), 131.41 (CH), 144.35 (C). Mass Spectrum (CI) mz: Found $(M + H)^+$, 133. Calculated for $C_8H_8N_2$, $(M + H)^+$, 133.

Example 238: 4-Aminomethyl benzonitrile was prepared according to example 236. The product was purified by distillation to give the product (65-85 % over two steps) as a colourless oil, which slowly solidifies, b.p. = 140°C at 0.25 mm Hg; $R_f$ = 0.37 (9:0.9:0.1 Dichloromethane:methanol:ammonia); 1H NMR ($CDCl_3$; 400 MHz) 1.519 (2H, br s, NH2), 3.96 (2H, s, CH2), 7.45 (2H, d, J 8.0, 2 x $H_m$), 7.62 (2H, d, J 8.0, 2 x $H_o$); 13C NMR ($CDCl_3$; 100 MHz) 45.866 (CH2), 110.217 (C), 118.786 (C), 127.457 (2 x CH), 132.033 (2 x CH), 148.267 (C); Mass Spectrum (CI) mz: Found $(M + H)^+$, 133. Calculated for $C_8H_8N_2$, $(M + H)^+$, 133.

Example 239: General procedure for the preparation of aminomethyl benzamidoximes. N,N-diisopropylethylamine (1.5 equivs) is added slowly dropwise to a stirred mixture of hydroxylamine hydrochloride (1.5 equiv) and the required aminomethyl benzonitrile (1.0 equiv) in dry ethanol, under nitrogen at room temperature. The resulting solution was then heated to reflux and followed by tlc, until there was no starting material left (normally 4 h). The reaction mixture was then cooled overnight, filtered, the white solid being washed with cold ethanol (30 ml) and then diisopropylether (2 x 30 ml). The filtrate and washing were evaporated down under reduced pressure to give crude reaction products. The products were purified by crystallisation from a methanol: diethyl ether mixture.

Example 240: 3-Aminomethyl benzamidoxime benzonitrile was prepared according to example 239. The compound was then dissolved in the minimum amount of methanol and then diethyl ether added, to give the product as a white powder. $R_f$ = 0.17 (4:0.9:0.1 dichloromethane:methanol:ammonia); 1H NMR (d6-DMSO; 360 MHz) 3.05 (3H, br s, OH + NH2), 3.735 (2H, s, CH2), 5.78 (2H, s, NH2), 7.65-7.28 (4H, m, Ar-H); 13C NMR (d6-DMSO;. 90 MHz) 45.532 (CH2), 123.299 (CH), 124.113 (CH), 127.554 (CH), 127.820 (CH), 133.089 (C), 143.844 (C), 150.963 (C); Mass Spectrum (EI) mz: Found $(M + H)^+$, 166. Calculated for $C_8H_{11}N_3O$, $(M + H)^+$, 166.

Example 241: 4-Aminomethyl benzamidoxime benzonitrile was prepared according to example 239. The compound was then dissolved in the minimum amount of methanol and then diethyl ether added, to give the product as a buff coloured solid. This solid can be recrystallised from iso-propanol to give the product as white powder. $R_f$ = 0.17 (4:0.9:0.1 dichloromethane : methanol : ammonia); 1H NMR ($CD_3OD$; 360 MHz) 3.799 (2H, s, CH2), 4.923 (5H, br s, 2 x NH2 + OH), 7.35 (2H, d, *J* 8.0, 2 x $H_m$), 7.59 (2H, d, *J* 8.0, 2 x $H_o$); 13C NMR ($CD_3OD$; 90 MHz) 46.082 (CH2), 127.165 (2 x CH), 128.188 (2 x CH), 132.498 (C), 145.094 (C), 155.082 (C) ; Mass Spectrum (EI) mz: Found $(M + H)^+$, 166. Calculated for $C_8H_{11}N_3O$, $(M + H)^+$, 166.

Example 242: General procedure for the preparation of azidomethyl benzamidoxime. N,N-diisopropylethylamine (1.5 equivs) is added dropwise, over 30 min, to a stirred mixture of hydroxylamine hydrochloride (1.5 equivs) and the required crude azidomethyl benzonitrile (1 equiv) in dry ethanol, under nitrogen at room temperature. The resulting solution is then heated to reflux and followed by tlc, until there was no starting material left (normally 3 h). The reaction solution is then cooled and evaporated, *in vacuo,* to give a crude product. The mixture is then partitioned between ethyl acetate and saturated sodium chloride solution. The ethyl acetate layer is separated and then the aqueous layer back extracted three times with ethyl acetate. The combined organic extracts are washed with saturated sodium chloride solution, dried ($Na_2SO_4$), and then evaporated down under, *in vacuo,* to give the crude product.

Example 243: Preparation of 3-azidomethyl benzamidoxime hydrochloride according to example 242. The crude compound was dissolved in the minimum amount of methanol and then cooled in an ice bath. To the brown solution was added hydrogen chloride (1 M in diethyl ether) to give a light brown solid. This was filtered and washed with diethyl ether, to give the product (81 % over two steps - from the bromo), as the hydrochloride salt, as a brown solid. $R_f$ 0.52 (ethyl acetate); 1H NMR ($CD_3OD$; 400 MHz) 4.552 (2H, s, CH2), 4.911 (3H, br s, NH2 + OH), 7.74-7.3

(4H, m, Ar-H); 13C NMR ($CD_3OD$; 100 MHz) 54.715 (CH2), 127.219 (C), 128.322 (CH), 128.411 (CH), 130.968 (CH), 134.314 (CH), 139.127 (C), 162.321 (C); Mass Spectrum (EI) mz: Found $(M + H)^+$, 192. Calculated for $C_8H_9N_5O$, $(M + H)^+$, 192.

Example 244: Preparation of 4-azidomethyl benzamidoxime hydrochloride according to example 242. The crude product was purified by recrystallisation from n-heptane:ethyl acetate (2:1) to give the compound (79 % over two steps - from the bromo) as white crystals. The nmr data on this product showed the compound to be at least 95% pure. $R_f$ 0.52 (ethyl acetate); 1H NMR (d6-DMSO; 400 MHz) 4.385 (2H, s, CH2), 5.802 (2H, s, NH2), 7.31 (2H, d, *J* 8.0, 2 x $H_m$), 7.66 (2H, d, *J* 8.0, 2 x $H_o$), 9.67 (1H, s, OH); 13C NMR (d6-DMSO; 100 MHz) 53.287 (CH2), 125.549 (2 x CH), 127.997 (2 x CH), 132.959 (C), 135.989 (C), 150.309 (C); Mass Spectrum (EI) mz: Found $(M + H)^+$, 192. Calculated for $C_8H_9N_5O$, $(M + H)^+$, 192.

Example 245: General procedure for the preparation aminomethyl benzamidines. The corresponding benzamidoxime is dissolved in 2N aqueous hydrochloric acid, and to this a catalytic amount of 10% Palladium on Carbon added. The resulting mixture is then saturated with hydrogen, by bubbling through the reaction mixture for 1 hr. The reaction is then left to hydrogenate under 1 atm of hydrogen, and followed by HPLC/MS. Occasionally, the reaction tends to stop after a certain time, and so the reaction mixture is filtered and fresh catalyst added, and again the is solution saturated with hydrogen and then stirred under a $H_2$ atmosphere until complete. When finished, the catalyst is filtered over Celite, and then washed twice with distilled water. The filtrate and washings are evaporated down under reduced pressure, to give the crude solid product. The products are then purified by dissolving in the minimum of methanol and adding diethyl ether, to give the products as white crystalline solids. These are collected and the solids washed with diethyl ether.

**Example** 246: Preparation of 3-aminomethyl benzamidine bis-hydrochloride according to example 245. White crystalline solid, yield = 75%. 1H NMR (d6-DMSO; 250 MHz) 4.09 (2H, s, CH2), 7.64-7.58 (1H, m, Ar-Hm), 7.87-7.84 (2H, m, Ar-$H_o$ + Ar-$H_p$), 8.14 (1H, s, Ar-$H_o$), 8.75 (3H, s), 9.37 (2H, s), 9.58 (2H, s); 13C NMR (d6-DMSO; 62.9 MHz) 42.186 (CH2), 128.181 (C), 128.329 (CH), 129.485 (CH), 129.762 (CH), 134.6141 (CH), 135.046 (C), 165.781 (C); Mass Spectrum (EI) mz: Found $(M + H)^+$, 150. Calculated for $C_8H_{11}N_3$, $(M + H)^+$, 150.

Example 247: Preparation of 4-aminomethyl benzamidine bis-hydrochloride according to example 245. White crystalline solid, yield = 55 - 87 %. 1H NMR (d6-DMSO; 400 MHz) 4.10 (2H, s, CH2), 7.73 (2H, d, *J* 8.0, 2 x $H_m$), 7.91 (2H, d, *J* 8.0, 2 x $H_o$), 8.863 (3H, s), 9.36 (2H, s), 9.633 (2H, s); 13C NMR (d6-DMSO; 100 MHz) 41.49 (CH2), 127.231 (C), 128.004 (2 x CH), 129.004 (2 x CH), 139.800 (C), 164.84 (C); Mass Spectrum (EI) mz: Found $(M + H)^+$, 150. Calculated for $C_8H_{11}N_3$, $(M + H)^+$, 150.

A compound of Formula (I), its solvate or salt can be used for the preparation of therapeutically useful agents as are e.g. tablets or capsules with the following composition:

| Example A | per tablet |
|---|---|
| biologically active compound | 150 mg |
| microcrystalline cellulose | 150 mg |
| starch | 25 mg |
| talc | 25 mg |
| hydroxypropylcellulose | 20 mg |
| | 370 mg |

| Example B | per capsule |
|---|---|
| biologically active compound | 100 mg |
| starch | 30 mg |
| lactose | 100 mg |
| talc | 5 mg |
| magnesium stearate | 1 mg |
| | 236 mg |

## Claims

**1.** Compounds of the Formula

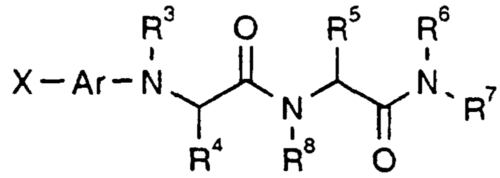

(I)wherein
X is $H_2N$-C(=NH)- or $R^1$-N=C(-$NH_2$)-, wherein
$R^1$ is -OH, -C(=O)O$R^2$, alkyl, aralkyl, aralkyloxy or a heteroalkyl group, such as alkyloxy, acyl or acyloxy, wherein
$R^2$ is alkyl, heteroalkyl, carbocyclic, heterocycloalkyl, aryl, heteroaryl or aralkyl;
Ar is arylene, heteroarylene, or aralkylene wherein X is directly attached to the aromatic ring system;
$R^3$ is H, alkyl, heteroalkyl or aralkyl;
$R^4$ is H, an alkyl group which may be substituted with one or more -OH or -$NH_2$ groups, a heteroalkyl group, a carbocyclic group, a heterocycloalkyl group, an aryl group, a heteroaryl group or an aralkyl group, which groups may be substituted with one or more groups selected from alkyl, heteroalkyl such as alkyloxy, acyl or acyloxy, a carbocyclic group, heterocycloalkyl, aryl, heteroaryl or aralkyl;
$R^5$ is H, alkyl, heteroalkyl, carbocyclic, heterocycloalkyl, aryl, heteroaryl or aralkyl;
$R^6$ and $R^7$ are independently H, alkyl, heteroalkyl, carbocyclic, heterocycloalkyl such as aryl-heterocycloalkyl, aryl, heteroaryl, aralkyl or heteroarylalkyl, which groups may be substituted with one or more groups selected from alkyl, heteroalkyl such as alkoxy, acyl or acyloxy, a carbocyclic group, heterocycloalkyl, aryl, heteroaryl, aralkyl, -OH or -$NH_2$,
or are members of a heterocycloalkyl ring system, in particular an aryl-heterocycloalkyl ring system, or a heteroaryl ring system, which systems may be substituted with one or more groups selected from alkyl, heteroalkyl such as alkoxy, acyl or acyloxy, a carbocyclic group, heterocycloalkyl, aryl, heteroaryl, aralkyl, -OH or -$NH_2$; and
$R^8$ is H, alkyl, heteroalkyl, carbocyclic, heterocycloalkyl, aryl, heteroaryl or aralkyl;
or a pharmacologically acceptable salt, solvate or hydrate thereof.

**2.** Compounds according to Claim 1, wherein
X is $H_2N$-C(=NH)- or $R^1$-N=C (-$NH_2$)-;
wherein $R^1$ is -OH or -C(=O)O$R^2$;
wherein $R^2$ is alkyl, heteroalkyl, carbocyclic, heterocycloalkyl, aryl, heteroaryl or aralkyl;
Ar is arylene, heteroarylene, or aralkylene;
$R^3$ is H, alkyl, heteroalkyl or aralkyl;
$R^4$ is H, alkyl which may be substituted with -OH or -$NH_2$ groups, heteroalkyl, carbocyclic, carboxyalkyl ester, heterocycloalkyl, aryl which may be substituted with acyl groups, heteroaryl or aralkyl;
$R^5$ is H, alkyl, heteroalkyl, carbocyclic, or aralkyl;
$R^6$ and $R^7$ are independently H, alkyl, heteroalkyl, carbocyclic, heterocycloalkyl, aryl, heteroaryl, arylheterocycloalkyl which may be substituted with acyl groups, heteroalkylaryl which may be substittued with alkyl groups, aralkyl which may be substituted with acyl groups, or are members of the same heteroalkyl, cycloalkyl, heterocycloalkyl, aryl, heteroaryl which may be substituted with alkylene groups, or aralkyl ring system, which may be substituted with -OH or -$NH_2$ groups; and
$R^8$ is H;
or a pharmaceutically acceptable salt, solvate or hydrate thereof.

**3.** Compounds according to Claim 1 or 2, wherein
X is $H_2N$-C(=NH)- or HO-N=C(-$NH_2$)- or $R^2$OC(=O)-N=C(-$NH_2$)-,
$R^3$ is H,
Ar is meta-phenylene, and
R is a $C_1$-$C_6$ alkyl or an aralkyl group.

**4.** Compounds according to Claims 1 to 3, wherein
X is $H_2N$-C(=NH)- or HO-N=C(-$NH_2$)- or $R^2$OC(=O)-N=C(-$NH_2$)-,
$R^3$ is H,

$R^4$ is H, methyl, hydroxymethyl, isopropyl, 2-imidazolyl, 3-pyrazolyl,
Ar is meta-phenylene,
$R^5$ is a $C_1$-$C_6$ alkyl or an aralkyl group, and
$R^8$ is H.

**5.** Compounds according to Claims 1 to 4, wherein
X is $H_2N$-C(=NH)- or HO-N=C(-$NH_2$)- or $R^2$OC(=O)-N=C(-$NH_2$)-,
$R^3$ is H,
$R^4$ is H, methyl, hydroxymethyl, 1,2-dihydroxyethyl, ethoxycarbonyl, isopropyl, cyclopropyl, 2-imidazolyl, 2-pyrrolyl, 3-pyrazolyl, 2-pyridyl, 4-methoxycarbonyl-phenyl,
Ar is meta-phenylene,
$R^5$ is a $C_1$-$C_6$ alkyl or an aralkyl group,
$R^6$ is H and $R^7$ is optionally substituted 1H-indol-3-yl-ethyl, 4-hydroxy-phenylethyl, cyclohexyl, N-(2-methoxyphenyl)piperazinyl, N-(4-methoxyphenyl) piperazinyl, 1,3-benzodioxol-5-ylmethyl, benzyl, phenethyl, 3,4-dimethoxyphenyl-1-ylmethyl, 2-methoxyphenyl-1-ylmethyl, 2-(4-morpholinyl)ethyl, 2-pyridinylethyl, 2-pyridinylpropyl, 3-pyridinylmethyl or $R^6$ and $R^7$ are part of a tetrahydroisoquinoline ring, a 4-thiomorpholine ring, a N-(2-methoxyphenyl) piperazine ring or a N-(4-methoxyphenyl)piperazine ring, and
$R^8$ is H

**6.** Compounds according to Claim 1, wherein
X is $H_2N$-C(=NH)- or HO-N=C(-$NH_2$)- or $R^2$OC(=O)-N=C (-$NH_2$)-,
$R^3$ is H,
Ar is para-phenylmethylene group, and
$R^5$ is a $C_1$-$C_6$ or an aralkyl group.

**7.** Compounds according to Claims 1 and 6, wherein
X is $H_2N$-C(=NH)- or HO-N=C(-$NH_2$)- or $R^2$OC(=O)-N=C(-$NH_2$)-,
$R^3$ is H,
$R^4$ is H, methyl, hydroxymethyl, isopropyl, 2-imidazolyl, 3-pyrazolyl,
Ar is para-phenylmethylene group, and
$R^5$ is a $C_1$-$C_6$ alkyl or an aralkyl group.

**8.** Compounds according to Claims 1, 6 and 7, wherein
X is $H_2N$-C(=NH)- or HO-N=C(-$NH_2$)- or $R^2$OC(=O)-N=C(-$NH_2$)-,
$R^3$ is H,
$R^4$ is H, methyl, hydroxymethyl, 1,2-dihydroxyethyl, ethoxycarbonyl, isopropyl, cyclopropyl, 2-imidazolyl, 2-pyrrolyl, 3-pyrazolyl, 3- or 4-phenoxy-phenyl, 1,3-benzodioxol-5-yl, 2-pyridyl, 4-methoxycarbonyl-phenyl,
Ar is para-phenylmethylene group,
$R^5$ is a $C_1$-$C_6$ alkyl or an aralkyl group,
$R^6$ is H and $R^7$ is optionally substituted 1H-indol-3-yl-ethyl, 4-hydroxy-phenethyl, cyclohexyl, N-(2-methoxyphenyl) piprazinyl, 1,3-benzodioxol-5-ylmethyl, benzyl, phenethyl, 3,4-dimethoxyphenyl-1-ylmethyl, 2-methoxyphenyl-1-ylmethyl, 2-(4-morpholinyl)ethyl, 2-pyridinylethyl, 2-pyridinylpropyl, 3-pyridinylmethyl or $R^6$ and $R^7$ are part of a tetrahydroisoquinoline ring, a 4-thiomorpholine ring, a N-(2-methoxyphenyl)piperazine ring or a N-(4-methoxyphenyl)piperazine ring, and
$R^8$ is H.

**9.** Pharmaceutical compositions containing a compound according to Claims 1 to 8 as the active agent and optionally carriers and/or adjuvants.

**10.** Pro-drugs, which are composed of a compound according to Claims 1 to 8 and at least one pharmacologically acceptable protective group which will be cleaved off under physiological conditions, selected from an alkoxy-, aralkyloxy-, acyl- or acyloxy group such as ethoxy, benzyloxy, acetyl or acetyloxy.

**11.** Process for the preparation of a compound according to Claims 1 to 8, wherein

a) a compound of Formula I, where X is a cyano group, is converted to a compound of Formula I, where X is a group of the Formula $H_2N$-C(=NH)- or $R^1$-N=C(-$NH_2$)-, and
b) this compound is optionally converted into a physiologically acceptable salt, solvate or hydrate.

**12.** Use of a compound, a pharmaceutical composition or a pro-drug according to claims 1 to 10 for the manufacture of medicaments for the inhibition of tryptase.

**13.** Use of a compound, a pharmaceutical composition or a pro-drug according to Claims 1 to 10 for the manufacture of medicaments for the treatment and/or prevention of diseases that are mediated by tryptase activity.

**14.** Use of a compound, a pharmaceutical composition or a pro-drug according to Claims 1 to 10 for the manufacture of medicaments for the treatment and/or prevention of allergic or inflammatory diseases.

**15.** Use of a compound, a pharmaceutical composition or a pro-drug according to Claims 1 to 10 for the manufacture of medicaments for the treatment and/or prevention of asthma, allergic rhinitis, chronic obstructive pulmonary diseases, emphysema, viral and bacterial pulmonary infections and inflammatory responses, rheumatoid arthritis, multiple sclerosis, osteoarthritis, dermatological diseases, psoriasis, conjunctivitis, inflammatory bowel diseases, peptic ulcers, cardiovascular diseases, anaphylaxis and cancer.

**16.** Use of a compound, a pharmaceutical composition or a pro-drug according to claims 1 to 10 for the manufacture of medicaments for the inhibition of factor Xa.

**Patentansprüche**

**1.** Verbindungen der Formel

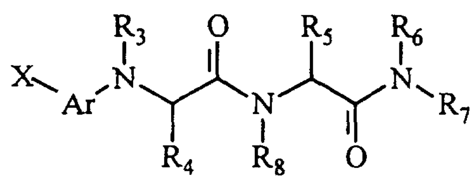

(I), worin
X $H_2N-C(=NH)-$ oder $R^1-N=C(-NH_2)-$ ist, worin
$R^1$ -OH, $-C(=O)OR^2$, Alkyl, Aralkyl, Aralkyloxy oder eine Heteroalkylgruppe, wie z.B. Alkyloxy, Acyl oder Acyloxy ist, worin
$R^2$ Alkyl, Heteroalkyl, carbozyklisch, Heterozykloalkyl, Aryl, Heteroaryl oder Aralkyl ist;
Ar Arylen, Heteroarylen, oder Aralkylen ist, worin X direkt an das aromatische Ringsystem gebunden ist;
$R^3$ H, Alkyl, Heteroalkyl oder Aralkyl ist;
$R^4$ H, eine Alkylgruppe, die mit einer oder mehreren -OH oder $-NH_2$ Gruppen substituiert sein kann, eine Heteroalkylgruppe, eine carbozyklische Gruppe, eine Heterozykloalkylgruppe, eine Arylgruppe, eine Heteroarylgruppe oder eine Aralkylgruppe ist, wobei diese Gruppen substituiert sein können mit einer oder mehreren Gruppen, ausgewählt aus Alkyl, Heteroalkyl, wie z.B.
Alkyloxy, Acyl oder Acyloxy, einer carbozyklischen Gruppe, Heterozykloalkyl, Aryl, Heteroaryl oder Aralkyl;
$R^5$ H, Alkyl, Heteroalkyl, carbozyklisch, Heterozykloalkyl, Aryl, Heteroaryl oder Aralkyl ist;
$R^6$ und $R^7$ eigenständig H, Alkyl, Heteroalkyl, carbozyklisch, Heterozykloalkyl sind, wie z.B. Arylheterozykloalkyl, Aryl, Heteroaryl, Aralkyl oder Heteroarylalkyl, wobei diese Guppen substituiert sein können mit einer oder mehreren Gruppen, ausgewählt aus Alkyl, Heteroalkyl, wie z.B. Alkoxy, Acyl oder Acyloxy, einer carbozyklischen Gruppe, Heterozykloalkyl, Aryl, Heteroaryl, Aralkyl, -OH oder $-NH_2$,
oder Teile von einem Heterozykloalkyl-Ringsystem, insbesondere einem Aryl-heterozykloalkyl-Ringsystem oder einem Heteroaryl-Ringsystem sind, wobei diese Systeme substituiert sein können mit einer oder mehreren Gruppen, ausgewählt aus Alkyl, Heteroalkyl, wie z.B. Alkoxy, Acyl oder Acyloxy, einer carbozyklischen Gruppe, Heterozykloalkyl, Aryl, Heteroaryl, Aralkyl, -OH oder $-NH_2$; und
$R^8$ H, Alkyl, Heteroalkyl, carbozyklisch, Heterozykloalkyl, Aryl, Heteroaryl oder Aralkyl ist;
oder ein pharmakologisch verträgliches Salz, Solvat oder Hydrat derselben.

**2.** Verbindungen nach Anspruch 1, worin
X $H_2N-C(=NH)-$ oder $R^1-N=C(-NH_2)$ - ist;

worin $R^1$ -OH oder -C(=O)OR$^2$ ist;
worin $R^2$ Alkyl, Heteroalkyl, carbozyklisch, Heterozykloalkyl, Aryl, Heteroaryl oder Aralkyl ist;
Ar Arylen, Heteroarylen oder Aralkylen ist;
$R^3$ H, Alkyl, Heteroalkyl oder Aralkyl ist;
$R^4$ H, Alkyl, das mit -OH oder -$NH_2$ Gruppen substituiert sein kann, Heteroalkyl, carbozyklisch, Carboxyalkylester, Heterozykloalkyl, Aryl, das mit Acylgruppen substituiert sein kann, Heteroaryl oder Aralkyl ist;
$R^5$ H, Alkyl, Heteroalkyl, carbozyklisch oder Aralkyl ist;
$R^6$ und $R^7$ eigenständig H, Alkyl, Heteroalkyl,
carbozyklisch, Heterozykloalkyl, Aryl, Heteroaryl, Arylheterozykloalkyl, das mit Acylgruppen substituiert sein kann, Heteroalkylaryl, das mit Alkylgruppen substituiert sein kann, Aralkyl, das mit Acylgruppen substituiert sein kann, sind oder Teile sind von dem gleichen Heteroalkyl, Zykloalkyl, Heterozykloalkyl, Aryl, Heteroaryl, das mit Alkylengruppen substituiert sein kann, oder von einem Aralkyl-Ringsystem, das mit-OH oder -$NH_2$ Gruppen substituiert sein kann; und
$R^8$ H ist;
oder ein pharmakologisch verträgliches Salz, Solvat oder Hydrat derselben.

**3.** Verbindungen nach Anspruch 1 oder 2, worin
X $H_2N$-C(=NH)- oder HO-N=C(-$NH_2$)- oder $R^2$0C(=O)-N=C(-$NH_2$)-,
$R^3$ H ist,
Ar meta-Phenylen ist, und
$R^5$ ein $C_1$-$C_6$ Alkyl oder eine Aralkylgruppe ist.

**4.** Verbindungen nach den Ansprüchen 1 bis 3, worin
X $H_2N$-C(=NH) - oder HO-N=C (-$NH_2$) - oder $R^2$0C (=O) -N=C (-$NH_2$)-,
$R^3$ H ist,
$R^4$ H, Methyl, Hydroxymethyl, Isopropyl, 2-Imidazolyl, 3-Pyrazolyl ist,
Ar meta-Phenylen ist,
$R^5$ ein $C_1$-$C_6$ Alkyl oder eine Aralkylgruppe ist, und
$R^8$ H ist.

**5.** Verbindungen nach den Ansprüchen 1 bis 4, worin
X $H_2N$-C(=NH)- oder HO-N=C(-$NH_2$)- oder $R^2$0C(=O) -N=C (-$NH_2$)-,
$R^3$ H ist,
$R^4$ H, Methyl, Hydroxymethyl, 1,2-Dihydroxyethyl, Ethoxycarbonyl, Isopropyl, Zyklopropyl, 2-Imidazolyl, 2-Pyrrolyl, 3-Pyrazolyl, 2-Pyridyl, 4-Methoxycarbonylphenyl ist,
Ar meta-Phenylen ist,
$R^5$ ein $C_1$-$C_6$ Alkyl oder eine Aralkylgruppe ist,
$R^6$ H ist und R7 wahlweise substituiert ist mit 1H-Indol-3-yl-ethyl, 4-Hydroxy-phenylethyl, Zyklohexyl, N-(2-Methoxyphenyl)piperazinyl, N-(4-Methoxyphenyl)piperazinyl, 1,3-Benzodioxol-5-ylmethyl, Benzyl, Phenethyl, 3,4-Dimethoxyphenyl-1-ylmethyl, 2-Methoxyphenyl-1-ylmethyl, 2-(4-Morpholinyl)ethyl, 2-Pyridinylethyl, 2-Pyridinylpropyl, 3-Pyridinylmethyl oder $R^6$ und $R^7$ Teil sind von einem Tetrahydroisoquinolinring, einem 4-Thiomorpholinring, einem N-(2-Methoxyphenyl)piperazinring oder einem N-(4-Methoxyphenyl)piperazinring, und
$R^8$ H ist.

**6.** Verbindungen nach Anspruch 1, worin
X $H_2N$-C(=NH)- oder HO-N=C(-$NH_2$)- oder $R^2$0C (=O) -N=C (-$NH_2$)-,
$R^3$ H ist,
Ar eine para-Phenylmethylengruppe ist, und
$R^5$ ein C1-C6 Alkyl oder eine Aralkylgruppe ist.

**7.** Verbindungen nach den Ansprüchen 1 bis 6, worin
X $H_2N$-C(=NH)- oder HO-N=C(-$NH_2$)- oder $R^2$0C(=O)-N=C(-$NH_2$)-, $R^3$ H ist,
$R^4$ H, Methyl, Hydroxymethyl, Isopropyl, 2-Imidazolyl, 3-Pyrazolyl ist,
Ar eine para-Phenylmethylengruppe ist, und
$R^5$ ein $C_1$-$C_6$ Alkyl oder eine Aralkylgruppe ist.

**8.** Verbindungen nach den Ansprüchen 1, 6 und 7, worin
X $H_2N$-C(=NH)- oder HO-N=C (-$NH_2$) - oder $R^2$0C (=O)-N=C (-$NH_2$)-ist,

$R^3$ H ist,
$R^4$ H, Methyl, Hydroxymethyl, 1,2-Dihydroxyethyl, Ethoxycarbonyl, Isopropyl, Zyklopropyl, 2-Imidazolyl, 2-Pyrrolyl, 3-Pyrazolyl, 3- oder 4-Phenoxy-phenyl, 1,3-Benzodioxol-5-yl, 2-Pyridyl, 4-Methoxycarbonyl-phenyl ist, Ar eine para-Phenylmethylengruppe ist,
$R^5$ ein $C_1$-$C_6$ Alkyl oder eine Aralkylgruppe ist,
$R^6$ H ist und R7 wahlweise substituiert ist mit 1H-Indol-3-yl-ethyl, 4-Hydroxy-phenethyl, Zyklohexyl, N-(2-Methoxyphenyl)piperazinyl, 1,3-Benzodioxol-5-ylmethyl, Benzyl, Phenethyl, 3,4-Dimethoxyphenyl-1-ylmethyl, 2-Methoxyphenyl-1-ylmethyl, 2-(4-Morpholinyl)ethyl, 2-Pyridinylethyl, 2-Pyridinylpropyl, 3-Pyridinylmethyl oder $R^6$ und $R^7$ Teil sind von einem Tetrahydroisoquinolinring, einem 4-Thiomorpholinring, einem N-(2-Methoxyphenyl)piperazinring oder einem N-(4-Methoxyphenyl)piperazinring, und
$R^8$ H ist.

**9.** Pharmazeutische Zusammensetzungen, enthaltend eine Verbindung gemäß den Ansprüchen 1 bis 8 als Wirkstoff und wahlweise Trägersubstanzen und/oder Zusatzstoffe.

**10.** Prodrugs, die zusammengesetzt sind aus einer Verbindung gemäß den Ansprüchen 1 bis 8 und wenigstens einer pharmakologisch verträglichen Schutzgruppe, die unter physiologischen Bedingungen abgespalten wird, ausgewählt aus einer Alkoxy-, Aralkyloxy-, Acyl- oder Acyloxygruppe, wie z.B. Ethoxy, Benzyloxy, Acetyl oder Acetyloxy.

**11.** Verfahren zur Herstellung einer Verbindung gemäß den Ansprüchen 1 bis 8, worin

a) eine Verbindung der Formel I, in der X eine Cyanogruppe ist, in eine Verbindung der Formel I umgewandelt wird, in der X eine Gruppe mit der Formel $H_2N$-C(=NH)- oder $R^1$-N=C(-$NH_2$) - ist, und
b) diese Verbindung wahlweise in ein physiologisch verträgliches Salz, Solvat oder Hydrat umgewandelt wird.

**12.** Verwendung einer Verbindung, einer pharmazeutischen Zusammensetzung oder einer Prodrug gemäß den Ansprüchen 1 bis 10 für die Herstellung von Medikamenten für die Hemmung von Tryptase.

**13.** Verwendung einer Verbindung, einer pharmazeutischen Zusammensetzung oder einer Prodrug gemäß den Ansprüchen 1 bis 10 für die Herstellung von Medikamenten für die Behandlung und/oder für die Vorbeugung von Krankheiten, die durch Tryptase-Aktivität vermittelt werden.

**14.** Verwendung einer Verbindung, einer pharmazeutischen Zusammensetzung oder einer Prodrug gemäß den Ansprüchen 1 bis 10 für die Herstellung von Medikamenten für die Behandlung und/oder für die Vorbeugung von Allergien oder Entzündungskrankheiten.

**15.** Verwendung einer Verbindung, einer pharmazeutischen Zusammensetzung oder einer Prodrug gemäß den Ansprüchen 1 bis 10 für die Herstellung von Medikamenten für die Behandlung und/oder für die Vorbeugung von Asthma, allergischer Rhinitis, chronischen Lungenverstopfungskrankheiten, Emphysem, viralen und bakteriellen Lungeninfektionen und entzündlichen Reaktionen, rheumatischer Arthritis, multipler Sklerose, Osteoarthritis, dermatologischen Krankheiten, Psoriasis, Konjunktivitis, Entzündungskrankheiten des Darms, Magengeschwüren, Kreislauferkrankungen, Anaphylaxie und Krebs.

**16.** Verwendung einer Verbindung, einer pharmazeutischen Zusammensetzung oder einer Prodrug gemäß den Ansprüchen 1 bis 10 für die Herstellung von Medikamenten für die Hemmung von Faktor Xa.

## Revendications

**1.** Composés de formule:

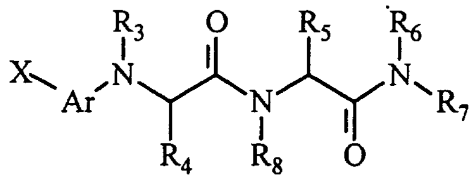

(I)

dans laquelle
X représente les groupements $H_2N-C(=NH)-$ ou $R^1-N=C(-NH_2)-$,
dans lesquels
$R^1$ représente un groupement -OH, $-C(=O)OR^2$, alkyle, aralkyle, aralkyloxy ou un groupe hétéroalkyle, tel que alkyloxy, acyle ou acyloxy,
dans lesquels
$R^2$ représente un groupement alkyle, hétéroalkyle, carbocyclique, hétérocycloalkyle, aryle, hétéroaryle, ou aralkyle;
Ar représente un groupe arylène, hétéroarylène ou aralkylène dans lequel X est directement lié au système cyclique aromatique ;
$R^3$ représente un atome d'hydrogène, un groupement alkyle, hétéroalkyle ou aralkyle;
$R^4$ représente un atome d'hydrogène, un groupement alkyle, pouvant porter un ou plusieurs substituants -OH ou $NH_2$, un groupe hétéroalkyle, un groupe carbocyclique, un groupe hétérocycloalkyle, un groupe aryle, un groupe hétéroaryle ou un groupe aralkyle, ces groupes pouvant porter un ou plusieurs substituants choisis parmi alkyle, hétéroalkyle tel que alkyloxy, acyle ou acyloxy, un groupe carbocyclique, hétérocycloalkyle, aryle, hétéroaryle ou aralkyle;
$R^5$ représente un atome d'hydrogène, un groupement alkyle, hétéroalkyle, carbocyclique, hétérocycloalkyle, aryle, hétéroaryle
ou aralkyle;
$R^6$ et $R^7$ représentent, indépendamment, un atome d'hydrogène, un groupement alkyle, hétéroalkyle, carbocyclique, hétérocycloalkyle tel que aryl-hétérocycloalkyle, aryle, hétéroaryle, aralkyle ou hétéroarylalkyle, ces groupes pouvant porter un ou plusieurs substituants choisis parmi alkyle, hétéroalkyle tel que alkoxy, acyle ou acyloxy, un groupe carbocyclique, hétérocycloalkyle, aryle, hétéroaryle, aralkyle, -OH ou $-NH_2$;
ou appartiennent à un système cyclique hétérocycloalkyle, en particulier un système cyclique aryl-hétérocycloalkyl, ou un système cyclique hétéroaryle, ces systèmes pouvant porter un ou plusieurs substituants choisis parmi alkyle, hétéroalkyle tel que alkoxy, acyle ou acyloxy, un groupe carbocyclique, hétérocycloalkyle, aryle, hétéroaryle, aralkyle, -OH ou $-NH_2$; et
$R^8$ représente un atome d'hydrogène, un groupe alkyle, hétéroalkyle, carbocyclique, hétérocycloalkyle, un groupe aryle, hétéroaryle ou aralkyle,
ou un solvate, un hydrate ou un sel pharmacologiquement acceptable de ceux-ci.

**2.** Composés selon la revendication 1, dans lesquels
X représente les groupements $H_2N-C(=NH)-$ ou $R^1-N=C(-NH_2)-$,
dans lesquels $R^1$ représente un groupement -OH ou $-C(=O)OR^2$,
dans lesquels $R^2$ représente un groupement alkyle, hétéroalkyle, carbocyclique, hétérocycloalkyle, aryle, hétéroaryle, ou aralkyle;
Ar représente un groupe arylène, hétéroarylène ou aralkylène;
$R^3$ représente un atome d'hydrogène, un groupement alkyle, hétéroalkyle ou aralkyle;
$R^4$ représente un atome d'hydrogène, un groupement alkyle, pouvant porter des substituants -OH ou $-NH_2$, un groupe hétéroalkyle, carbocyclique, ester de carboxyalkyle, hétérocycloalkyle, aryle pouvant porter des substituants acyle, hétéroaryle ou aralkyle ;
$R^5$ représente un atome d'hydrogène, un groupe alkyle, hétéroalkyle, carbocyclique, ou aralkyle;
$R^6$ et $R^7$ représentent, indépendamment, un atome d'hydrogène, un groupement alkyle, un groupe hétéroalkyle, carbocyclique, hétérocycloalkyle, aryle, hétéroaryle, arylhétérocycloalkyle pouvant porter des substituants acyle,

hétéroalkylaryle pouvant porter des substituants alkyle, aralkyle pouvant porter des substituants acyle, ou appartenant à un même hétéroalkyle, cycloalkyle, hétérocycloalkyle, aryle, hétéroaryle pouvant porter des substituants alkylène, ou un système cyclique aralkyle pouvant porter des substituants -OH et $-NH_2$; et
$R^8$ représente un atome d'hydrogène ;
ou un solvate, un hydrate ou un sel pharmacologiquement acceptable de ceux-ci.

**3.** Composés selon la revendication 1 ou 2, dans lesquels
X représente les groupements $H_2N-C(=NH)-$ ou $HO-N=C(-NH_2)-$ ou
$R^2OC(=O)-N=C(-NH_2)-$;
$R^3$ représente un atome d'hydrogène ;
Ar représente un groupe méta-phénylène; et
$R^5$ représente un groupe alkyle en $C_1$-$C_6$ ou un groupe aralkyle.

**4.** Composés selon les revendications 1 à 3, dans lesquels
X représente les groupements $H_2N-C(=NH)-$ ou $HO-N=C(-NH_2)-$ ou
$R^2OC(=O)-N=C(-NH_2)-$;
$R^3$ représente un atome d'hydrogène ;
$R^4$ représente un atome d'hydrogène, un méthyle, hydroxyméthyle, isopropyle, 2-imidazolyle, 3-pyrazolyle;
Ar représente un groupe méta-phénylène;
$R^5$ représente un groupe alkyle en $C_1$-$C_6$ ou un groupe aralkyle; et
$R^8$ représente un atome d'hydrogène.

**5.** Composés selon les revendications 1 à 4, dans lesquels
X représente les groupements $H_2N-C(=NH)-$ ou $HO-N=C(-NH_2)-$ ou
$R^2OC(=O)-N=C(-NH_2)-$;
$R^3$ représente un atome d'hydrogène ;
$R^4$ représente un atome d'hydrogène, un méthyle, hydroxyméthyle, 1,2-dihydroxyéthyle, éthoxycarbonyle, isopropyle, cyclopropyle, 2-imidazolyle, 2-pyrrolyle, 3-pyrazolyle, 2-pyridyle, 4-méthoxycarbonylphényle;
Ar représente un groupe méta-phénylène;
$R^5$ représente un groupe alkyle en $C_1$-$C_6$ ou un groupe aralkyle;
$R^6$ représente un atome d'hydrogène et $R^7$ représente un groupe éventuellement substitué choisi parmi 1H-indol-3-yl-éthyl, 4-hydroxy-phénéthyle, cyclohéxyle, N-(2-méthoxyphényl)pipérazinyle, N-(4-méthoxyphényl)pipérazinyle, 1,3-benzodioxol-5-ylméthyle, benzyle, phénéthyle, 3,4-diméthoxyphényl-1-ylméthyle, 2-méthoxyphényl-1-ylméthyle, 2-(4-morpholinyl)éthyle, 2-pyridinyléthyle, 2-pyridinylpropyle, 3-pyridinylméthyle, ou $R^6$ et $R^7$ représentent une partie d'un cycle tétrahydroisoquinoline, un cycle 4-thiomorpholine, un cycle N-(2-méthoxyphényl)pipérazine, ou un cycle N-(4-méthoxyphényl)pipérazine, et
$R^8$ représente un atome d'hydrogène.

**6.** Composés selon la revendication 1, dans lesquels
X représente les groupements $H_2N-C(=NH)-$ ou $HO-N=C(-NH_2)-$ ou
$R^2OC(=O)-N=C(-NH_2)-$;
$R^3$ représente un atome d'hydrogène
Ar représente un groupe para-phénylméthylène; et
$R^5$ représente un groupe alkyle en $C_1$-$C_6$ ou un groupe aralkyle.

**7.** Composés selon les revendications 1 et 6, dans lesquels
X représente les groupements $H_2N-C(=NH)-$ ou $HO-N=C(-NH_2)-$ ou
$R^2OC(=O)-N=C(-NH_2)-$;
$R^3$ représente un atome d'hydrogène ;
$R^4$ représente un atome d'hydrogène, méthyle, hydroxyméthyle, isopropyle, 2-imidazolyle, 3-pyrazolyle;
Ar représente un groupe para-phénylméthylène; et
$R^5$ représente un groupe alkyle en $C_1$-$C_6$ ou un groupe aralkyle.

**8.** Composés selon les revendications 1, 6 et 7, dans lesquels
X représente les groupements $H_2N-C(=NH)-$ ou $HO-N=C(-NH_2)-$ ou
$R^2OC(=O)-N=C(-NH_2)-$;
$R^3$ représente un atome d'hydrogène ;
$R^4$ représente un atome d'hydrogène, méthyle, hydroxyméthyle, 1,2-dihydroxyéthyle, éthoxycarbonyle, isopropyle,

cyclopropyle, 2-imidazolyle, 2-pyrrolyle, 3-pyrazolyle, 3- ou 4-phénoxy-phényle, 1,3-benzodioxol-5-yle, 2-pyridyle, 4-méthoxycarbonyl-phényle;
Ar représente un groupe para-phénylméthylène;
$R^5$ représente un groupe alkyle en $C_1$-$C_6$ ou un groupe aralkyle;
$R^6$ représente un atome d'hydrogène et $R^7$ représente un groupe éventuellement substitué choisi parmi 1H-indol-3-yl-éthyle, 4-hydroxy-phénéthyle, cyclohéxyle, N-(2-méthoxyphényl)pipérazinyle, 1,3-benzodioxol-5-ylméthyle, benzyle, phénéthyle, 3,4-diméthoxyphényl-1-ylméthyle, 2-méthoxyphényl-1-ylméthyle, 2-(4-morpholinyl)éthyle, 2-pyridinyléthyle, 2-pyridinylpropyle, 3-pyridinylméthyle, ou $R^6$ et $R^7$ représentent une partie du cycle tétrahydroisoquinoline, un cycle 4-thiomorpholine, un cycle N-(2-méthoxyphényl)pipérazine,
ou un cycle N-(4-méthoxyphényl)pipérazine, et
$R^8$ représente un atome d'hydrogène.

**9.** Compositions pharmaceutiques contenant un composé selon les revendications 1 à 8 en tant que principe actif et éventuellement support et/ou adjuvants.

**10.** Promédicaments, constitués de composés selon les revendications 1 à 8 et au moins un groupe protecteur pharmacologiquement acceptable, qui sera retiré sous des conditions physiologiques, choisi parmi les groupes alkoxy, aralkyloxy, acyle ou acyloxy, tels que éthoxy, benzyloxy, acétyle ou acétyloxy.

**11.** Procédé pour la préparation d'un composé selon les revendications 1 à 8, dans lequel :

a) le composé de formule I, dans lequel X représente un groupe cyano est transformé en un composé de formule I, dans lequel le groupe X a pour formule $H_2N$-C(=NH)- ou $R^1$-N=C(-$NH_2$)-, et
b) ce composé est éventuellement converti en un solvate, un hydrate ou un sel physiologiquement acceptable.

**12.** Utilisation d'un composé, d'une composition pharmaceutique ou d'un promédicament selon les revendications 1 à 10 pour la fabrication de médicaments destinés à l'inhibition de la tryptase.

**13.** Utilisation d'un composé, d'une composition pharmaceutique ou d'un promédicament selon les revendications 1 à 10 pour la fabrication de médicaments pour le traitement et/ou la prévention de maladies liées à l'activité de la tryptase.

**14.** Utilisation d'un composé, d'une composition pharmaceutique ou d'un promédicament selon les revendications 1 à 10 pour la fabrication de médicaments pour le traitement et/ou la prévention de maladies allergiques ou inflammatoires.

**15.** Utilisation d'un composé, d'une composition pharmaceutique ou d'un promédicament selon les revendications 1 à 10 pour la fabrication de médicaments pour le traitement et/ou la prévention de l'asthme, rhinite allergique, maladies d'obstruction pulmonaires chroniques, emphysème, infections pulmonaires virales et bactériennes et réactions inflammatoires, arthrite rhumatoïde, sclérose en plaque, ostéo-arthrite, maladies dermatologiques, psoriasis, conjonctivites, affections abdominales inflammatoires, ulcères gastriques, maladies cardiovasculaires, anaphylaxie et cancer.

**16.** Utilisation d'un composé, d'une composition pharmaceutique ou d'un promédicament selon les revendications 1 à 10 pour la fabrication de médicaments destinés à l'inhibition du facteur Xa.